(19) Europäisches Patentamt
European Patent Office
Office européen des brevets

(11) EP 3 521 446 A1

(12) EUROPEAN PATENT APPLICATION
published in accordance with Art. 153(4) EPC

(43) Date of publication:
**07.08.2019 Bulletin 2019/32**

(21) Application number: **17855651.0**

(22) Date of filing: **07.09.2017**

(51) Int Cl.:
***C12Q 1/68*** *(2018.01)* ***C12N 15/09*** *(2006.01)*

(86) International application number:
**PCT/JP2017/032252**

(87) International publication number:
**WO 2018/061693 (05.04.2018 Gazette 2018/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.09.2016 JP 2016192242**

(71) Applicant: **FUJIFILM Corporation**
**Tokyo 106-8620 (JP)**

(72) Inventor: **TSUJIMOTO, Takayuki**
**Ashigara-kami-gun**
**Kanagawa 258-8538 (JP)**

(74) Representative: **Klunker IP Patentanwälte PartG mbB**
**Destouchesstraße 68**
**80796 München (DE)**

# (54) METHOD FOR DESIGNING PRIMER FOR MULTIPLEX PCR

(57) There is provided a method for designing primers for multiplex PCR, in which amplification variations for each region of interest can be suppressed in a plurality of single cells by repeatedly attempting multiplex PCR for a small number of regions of interest, separating the regions of interest into a group of regions of interest for which the coefficient of variation for the number of sequence reads is greater than or equal to a threshold value and a group of regions of interest for which the coefficient of variation for the number of sequence reads is less than the threshold value, generating a histogram for each group, each histogram having a horizontal axis representing the average Tm value of a pair of primers used to PCR amplify each region of interest and a vertical axis representing the number of regions of interest, calculating the lower limit value and the upper limit value of a Tm value range for primer design, setting the obtained Tm value range, and designing primers.

FIG. 1

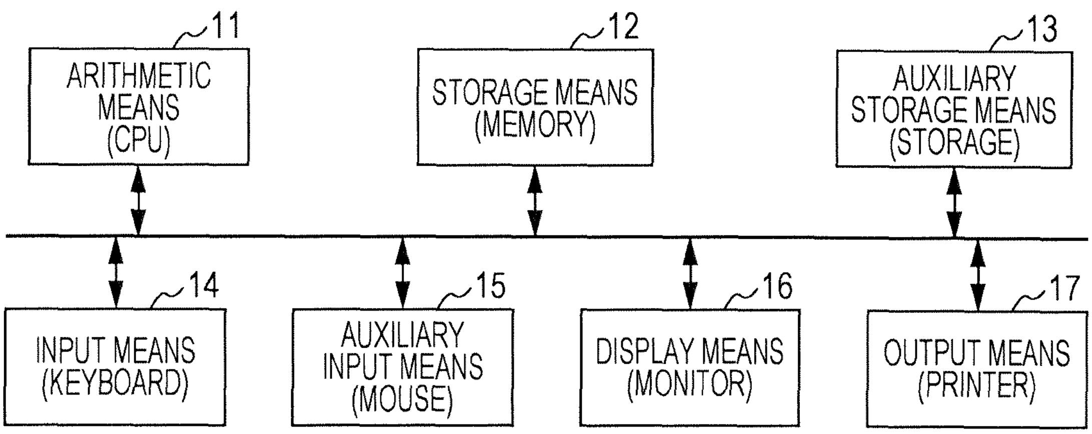




Printed by Jouve, 75001 PARIS (FR)

SEQUENCE LISTING

<110> Fuji Film Corporation

<120> Method for designing primers used in multiplex PCR

<130> W-6024PCT

<150> JP2016-192242
<151> 2016-09-29

<160> 78

<170> PatentIn version 3.5

<210> 1
<211> 20
<212> DNA
<213> Artificial Sequence

<220>
<223> V01-F

<400> 1
cttcgatgcg gaccttctgg 20

<210> 2
<211> 20
<212> DNA
<213> Artificial Sequence

<220>
<223> V01-R

<400> 2
tctcccacat ccggctatgg 20

<210> 3
<211> 20
<212> DNA
<213> Artificial Sequence

<220>
<223> V02-F

<400> 3
ggagacttct ctgagtctgg 20

<210> 4
<211> 20
<212> DNA
<213> Artificial Sequence

<220>
<223> V02-R

<400> 4
acacgttcaa gagggtttgg 20

<210> 5
<211> 20
<212> DNA
<213> Artificial Sequence

<220>
<223> V03-F

<400> 5
cctctgcaga gcttccttgg 20

<210> 6
<211> 20
<212> DNA
<213> Artificial Sequence

<220>
<223> V03-R

<400> 6
cacggttctc ctgtacttgg 20

<210> 7
<211> 20
<212> DNA
<213> Artificial Sequence

<220>
<223> V04-F

<400> 7
agttcagcgc tgaagcttgg 20

<210> 8
<211> 20
<212> DNA
<213> Artificial Sequence

<220>
<223> V04-R

<400> 8
cttgtttagg agagcgttgg 20

<210> 9
<211> 20
<212> DNA
<213> Artificial Sequence

<220>
<223> V05-F

<400> 9
tttagcttca ctgagcttgg 20

<210> 10
<211> 20
<212> DNA

<213> Artificial Sequence

<220>
<223> V05-R

<400> 10
ctcggtggtt ctgctgttgg 20

<210> 11
<211> 20
<212> DNA
<213> Artificial Sequence

<220>
<223> V06-F

<400> 11
cactgttgag tagagagtgg 20

<210> 12
<211> 20
<212> DNA
<213> Artificial Sequence

<220>
<223> V06-R

<400> 12
ttcgcttaat ctttggctgg 20

<210> 13
<211> 20
<212> DNA
<213> Artificial Sequence

<220>
<223> V07-F

<400> 13
tcgaaatggc atgtgtctgg 20

<210> 14
<211> 20
<212> DNA
<213> Artificial Sequence

<220>
<223> V07-R

<400> 14
gcctaagaat tacccggtgg 20

<210> 15
<211> 20
<212> DNA
<213> Artificial Sequence

<220>

<223> V08-F

<400> 15
tggataggct ggatcagtgg 20

<210> 16
<211> 20
<212> DNA
<213> Artificial Sequence

<220>
<223> V08-R

<400> 16
gaaccacagt caggagatgg 20

<210> 17
<211> 20
<212> DNA
<213> Artificial Sequence

<220>
<223> V09-F

<400> 17
atcagcagga ctgtgcatgg 20

<210> 18
<211> 20
<212> DNA
<213> Artificial Sequence

<220>
<223> V09-R

<400> 18
tacaacctgg cttagaatgg 20

<210> 19
<211> 20
<212> DNA
<213> Artificial Sequence

<220>
<223> V10-F

<400> 19
gtgccttctc ttcgttctgg 20

<210> 20
<211> 20
<212> DNA
<213> Artificial Sequence

<220>
<223> V10-R

<400> 20

tgacccgctt gtgtcaatgg 20

<210> 21
<211> 20
<212> DNA
<213> Artificial Sequence

<220>
<223> V11-F

<400> 21
tggcccctac ttaaatctgg 20

<210> 22
<211> 20
<212> DNA
<213> Artificial Sequence

<220>
<223> V11-R

<400> 22
tgctggagcg agtagactgg 20

<210> 23
<211> 20
<212> DNA
<213> Artificial Sequence

<220>
<223> V12-F

<400> 23
ctgagtaagt tcaggattgg 20

<210> 24
<211> 20
<212> DNA
<213> Artificial Sequence

<220>
<223> V12-R

<400> 24
ttcagttatc agtgcagtgg 20

<210> 25
<211> 20
<212> DNA
<213> Artificial Sequence

<220>
<223> V13-F

<400> 25
agtcccagca ctctctgtgg 20

<210> 26
<211> 20
<212> DNA
<213> Artificial Sequence

<220>
<223> V13-R

<400> 26
cccactggga tgctaactgg 20

<210> 27
<211> 20
<212> DNA
<213> Artificial Sequence

<220>
<223> V14-F

<400> 27
gaaaggaacg tgttgagtgg 20

<210> 28
<211> 20
<212> DNA
<213> Artificial Sequence

<220>
<223> V14-R

<400> 28
cccctcatga tttaagatgg 20

<210> 29
<211> 20
<212> DNA
<213> Artificial Sequence

<220>
<223> V15-F

<400> 29
ggaagcatcc aaggaagtgg 20

<210> 30
<211> 20
<212> DNA
<213> Artificial Sequence

<220>
<223> V15-R

<400> 30
agcacatgca gtgcctgtgg 20

<210> 31
<211> 20
<212> DNA

<213>  Artificial Sequence

<220>
<223>  V16-F

<400>  31
ccactaccac taggggatgg                                                     20

<210>  32
<211>  20
<212>  DNA
<213>  Artificial Sequence

<220>
<223>  V16-R

<400>  32
tagctgccaa agactgttgg                                                     20

<210>  33
<211>  20
<212>  DNA
<213>  Artificial Sequence

<220>
<223>  V17-F

<400>  33
aacagtgaat ggtgcattgg                                                     20

<210>  34
<211>  20
<212>  DNA
<213>  Artificial Sequence

<220>
<223>  V17-R

<400>  34
agtcttgagc gtgttagtgg                                                     20

<210>  35
<211>  20
<212>  DNA
<213>  Artificial Sequence

<220>
<223>  V18-F

<400>  35
ctcaccaaag ctgagactgg                                                     20

<210>  36
<211>  20
<212>  DNA
<213>  Artificial Sequence

<220>

<223> V18-R

<400> 36
tgcctgttgg gttttgctgg 20

<210> 37
<211> 20
<212> DNA
<213> Artificial Sequence

<220>
<223> V19-F

<400> 37
gcaacactaa cataggatgg 20

<210> 38
<211> 20
<212> DNA
<213> Artificial Sequence

<220>
<223> V19-R

<400> 38
tgacttctgc gcaaatttgg 20

<210> 39
<211> 20
<212> DNA
<213> Artificial Sequence

<220>
<223> V20-F

<400> 39
ttgtaggagc ctgggcttgg 20

<210> 40
<211> 20
<212> DNA
<213> Artificial Sequence

<220>
<223> V20-R

<400> 40
ggggaaacac tatgaagtgg 20

<210> 41
<211> 20
<212> DNA
<213> Artificial Sequence

<220>
<223> V21-F

<400> 41

tttccccgac cataagcttg 20

<210> 42
<211> 20
<212> DNA
<213> Artificial Sequence

<220>
<223> V21-R

<400> 42
atacagggct gagagattgg 20

<210> 43
<211> 20
<212> DNA
<213> Artificial Sequence

<220>
<223> V22-F

<400> 43
tgataaggtc cgaactttgg 20

<210> 44
<211> 20
<212> DNA
<213> Artificial Sequence

<220>
<223> V22-R

<400> 44
gcgactgcaa gagattcgtg 20

<210> 45
<211> 20
<212> DNA
<213> Artificial Sequence

<220>
<223> V23-F

<400> 45
atttgctgct gaccagggtg 20

<210> 46
<211> 20
<212> DNA
<213> Artificial Sequence

<220>
<223> V23-R

<400> 46
aggtacagct tcccatctgg 20

<210> 47
<211> 20
<212> DNA
<213> Artificial Sequence

<220>
<223> V24-F

<400> 47
ccgtgtgtga gattctcgtg 20

<210> 48
<211> 20
<212> DNA
<213> Artificial Sequence

<220>
<223> V24-R

<400> 48
actgctcagg gtcctctgtg 20

<210> 49
<211> 20
<212> DNA
<213> Artificial Sequence

<220>
<223> V25-F

<400> 49
gtaaagcctc caggatgttg 20

<210> 50
<211> 20
<212> DNA
<213> Artificial Sequence

<220>
<223> V25-R

<400> 50
ctggcacttg tgctgactgg 20

<210> 51
<211> 20
<212> DNA
<213> Artificial Sequence

<220>
<223> V26-F

<400> 51
ccaaagcgca ctcacctgtg 20

<210> 52
<211> 20
<212> DNA

<213>  Artificial Sequence

<220>
<223>  V26-R

<400>  52
tagccagtga gagcgaagtg 20

<210>  53
<211>  20
<212>  DNA
<213>  Artificial Sequence

<220>
<223>  V27-F

<400>  53
ggcctagagg acgatgcttg 20

<210>  54
<211>  20
<212>  DNA
<213>  Artificial Sequence

<220>
<223>  V27-R

<400>  54
tgttgataac catgccggtg 20

<210>  55
<211>  20
<212>  DNA
<213>  Artificial Sequence

<220>
<223>  V28-F

<400>  55
tgctggacag tgactcatgg 20

<210>  56
<211>  20
<212>  DNA
<213>  Artificial Sequence

<220>
<223>  V28-R

<400>  56
cattttcctg tcctggcttg 20

<210>  57
<211>  20
<212>  DNA
<213>  Artificial Sequence

<220>

<223> V29-F

<400> 57
atccagttca tatgccgttg 20

<210> 58
<211> 20
<212> DNA
<213> Artificial Sequence

<220>
<223> V29-R

<400> 58
gcgttgctgt cattcctttg 20

<210> 59
<211> 20
<212> DNA
<213> Artificial Sequence

<220>
<223> V30-F

<400> 59
cctggcggtt gacttctttg 20

<210> 60
<211> 20
<212> DNA
<213> Artificial Sequence

<220>
<223> V30-R

<400> 60
aatttgttga gatgcggttg 20

<210> 61
<211> 20
<212> DNA
<213> Artificial Sequence

<220>
<223> V31-F

<400> 61
agaagcaggt gaagatctgg 20

<210> 62
<211> 20
<212> DNA
<213> Artificial Sequence

<220>
<223> V31-R

<400> 62

cgtcatcctc ggagcacttg 20

<210> 63
<211> 20
<212> DNA
<213> Artificial Sequence

<220>
<223> V32-F

<400> 63
agagtccacg ctcctcatgg 20

<210> 64
<211> 20
<212> DNA
<213> Artificial Sequence

<220>
<223> V32-R

<400> 64
cagagccctt gagtccggtg 20

<210> 65
<211> 20
<212> DNA
<213> Artificial Sequence

<220>
<223> V33-F

<400> 65
agaccacacg tcgctcttgg 20

<210> 66
<211> 20
<212> DNA
<213> Artificial Sequence

<220>
<223> V33-R

<400> 66
ggacactcgg gttgaatgtg 20

<210> 67
<211> 20
<212> DNA
<213> Artificial Sequence

<220>
<223> V34-F

<400> 67
gagagaacaa gacggaggtg 20

<210> 68
<211> 20
<212> DNA
<213> Artificial Sequence

<220>
<223> V34-R

<400> 68
ggaggggtgc tggaatattg 20

<210> 69
<211> 20
<212> DNA
<213> Artificial Sequence

<220>
<223> V35-F

<400> 69
agatgacggc agtaggattg 20

<210> 70
<211> 20
<212> DNA
<213> Artificial Sequence

<220>
<223> V35-R

<400> 70
agagatgcct tcagaactgg 20

<210> 71
<211> 20
<212> DNA
<213> Artificial Sequence

<220>
<223> V36-F

<400> 71
actggcctag tgttcctgtg 20

<210> 72
<211> 20
<212> DNA
<213> Artificial Sequence

<220>
<223> V36-R

<400> 72
gtcacaatgc tggacgatgg 20

<210> 73
<211> 20
<212> DNA

<213>  Artificial Sequence

<220>
<223>  V37-F

<400>  73
atttggccct agccctcgtg                                                    20

<210>  74
<211>  20
<212>  DNA
<213>  Artificial Sequence

<220>
<223>  V37-R

<400>  74
attcacagcg aaagcagtgg                                                    20

<210>  75
<211>  20
<212>  DNA
<213>  Artificial Sequence

<220>
<223>  V38-F

<400>  75
gagccacgta tgttggggtg                                                    20

<210>  76
<211>  20
<212>  DNA
<213>  Artificial Sequence

<220>
<223>  V38-R

<400>  76
aaagggcttt tgagctcttg                                                    20

<210>  77
<211>  20
<212>  DNA
<213>  Artificial Sequence

<220>
<223>  V39-F

<400>  77
cctgtttccc atccaacgtg                                                    20

<210>  78
<211>  20
<212>  DNA
<213>  Artificial Sequence

<220>

<223>  V39-R

<400>  78
gtcaccatca tcagaagtgg                                                    20

## Description

## BACKGROUND OF THE INVENTION

1. Field of the Invention

The present invention relates to a method for designing primers for multiplex PCR.

2. Description of the Related Art

DNA sequencers and the like, which have been developed in recent years, facilitate genetic analysis. However, the total base length of the genome is generally enormous, and, on the other hand, sequencers have limited reading capacity. Accordingly, a PCR method is spreading as a technique for efficient and accurate genetic analysis by amplifying only a necessary specific gene region and reading only its base sequence. In particular, a method for selectively amplifying a plurality of gene regions by simultaneously supplying a plurality of types of primers to a certain single PCR reaction system is referred to as multiplex PCR.

JP5079694B describes a method for designing primers to be used in multiplex PCR for m regions of interest that are arranged on the same chromosome in coordinate order. Here, m is an integer greater than or equal to 1.

First, candidate primers corresponding to a first region of interest $X_1$ from a base sequence of DNA to be amplified are selected. At this time, candidate primers are assumed to be selected based on the primer melting temperature Tm, the GC content, the base sequence length, the base sequence specificity, and the score indicating the unlikelihood of formation of a hairpin structure and a primer dimer. Among n candidate primers corresponding to the region of interest $X_1$ for which the melting temperature, the GC content, and the base sequence length fall within predetermined ranges, the candidate having the highest score, where the score indicates the superiority of the candidate primer and is calculated from the base sequence specificity and the unlikelihood of formation of a hairpin structure and a primer dimer, is referred to as $P_{11}$, the candidate having the second highest score is referred to as $P_{12}$, and the n-th candidate is referred to as $P_{1n}$. Also, n' candidate primers $P_{21}$, $P_{22}$, ..., and $P_{2n'}$ corresponding to a second region of interest $X_2$ are selected in a manner similar to that described above. Similar operations are repeated for all the regions of interest to select k candidate primers $P_{m1}$, $P_{m2}$, ..., and $P_{mk}$ corresponding to the m-th region of interest $X_m$.

Then, to select a combination of primers that are optimum for a reaction from the selected candidate primers, whether primers in different regions of interest have no complementary base sequences at unintended sites is examined. Primers that are less likely to form a primer dimer among primers in different regions of interest are primers available for multiplex PCR.

## SUMMARY OF THE INVENTION

However, if multiplex PCR is performed on, in particular, as minute an amount of genomic DNA as several pg to ten and several pg, which is extracted from a single cell to amplify several hundreds of regions or more, even for the same region, regions with large amplification variations in a plurality of single cells may be present.

The presence of regions with large amplification variations in a plurality of single cells for each region implies unstable DNA amplification. It is thus desirable to reduce amplification variations in a plurality of single cells to enable more stable DNA amplification.

Accordingly, it is an object of the present invention to provide a method for designing primers for multiplex PCR, in which amplification variations for each region of interest can be suppressed in a plurality of single cells.

As a result of intensive studies to solve the problems described above, the present inventor has found that amplification variations for each region of interest can be suppressed in a plurality of single cells by: repeatedly attempting multiplex PCR for a small number of regions of interest; separating the regions of interest into a group of regions of interest for which the coefficient of variation for the number of sequence reads is greater than or equal to a threshold value and a group of regions of interest for which the coefficient of variation for the number of sequence reads is less than the threshold value; generating a histogram for each group, each histogram having a horizontal axis representing the average Tm value of a pair of primers used to PCR amplify each region of interest and a vertical axis representing the number of regions of interest; calculating the lower limit value and the upper limit value of a Tm value range for primer design; setting the obtained Tm value range; and designing primers. Finally, the present inventor has accomplished the present invention.

[1] A method for designing primers for multiplex PCR from a single cell, including a Tm value range setting step of setting a Tm value range for primer design, wherein

in a case where an attempt to PCR amplify m regions of interest out of n regions of interest and to count the number of sequence reads in each region of interest is made N times to calculate a coefficient of variation for the number of sequence reads in each region of interest, given that an actual value of a coefficient of variation for the number of sequence reads in an i-th region of interest is denoted by $CV_i$ and an average Tm value of a pair of primers used to PCR amplify the i-th region of interest is denoted by $Tm_i$,
in the Tm value range setting step, a Tm value range of a primer is determined by:

a step of inputting a target value $CV_0$ of coefficients of variation for the numbers of sequence reads from input means, and storing the target value $CV_0$ in storage means;
a step of inputting the number of regions of interest m in the attempt made N times, the actual value $CV_i$ of the coefficient of variation for the number of sequence reads in the i-th region of interest, and the average Tm value $Tm_i$ of the pair of primers used to PCR amplify the i-th region of interest, and storing the number of regions of interest m, the actual value $CV_i$, and the average Tm value $Tm_i$ in the storage means;
a step of, by arithmetic means, calculating a threshold value $CV_t$ for the coefficients of variation for the numbers of sequence reads as a function of the target value $CV_0$ in accordance with $CV_t = H(CV_0)$, and storing the threshold value $CV_t$ in the storage means;
a step of, by the arithmetic means, separating the m regions of interest into an R1 group constituted by $m_1$ regions of interest in which the coefficient of variation $CV_i$ for the number of sequence reads satisfies $CV_i \geq CV_t$ and an R2 group constituted by $m_2$ regions of interest in which the coefficient of variation $CV_i$ for the number of sequence reads satisfies $CV_i < CV_t$, generating respective histograms for the R1 group and the R2 group, each histogram having a horizontal axis representing an average Tm value of a pair of primers used to PCR amplify each region of interest and a vertical axis representing the number of regions of interest, and storing the histograms in the storage means;
a step of, by the arithmetic means, calculating a value designated in advance from a value at a left end of the histogram for the R1 group, a value at a right end of the histogram for the R1 group, a mode of the histogram for the R1 group, a value at a left end of the histogram for the R2 group, and a Tm value at an intersection of the histogram for the R1 group and the histogram for the R2 group, and storing the calculated value as a lower limit value of the Tm value range in the storage means;
a step of, by the arithmetic means, calculating a value at a right end of the histogram for the R2 group, and storing the calculated value as an upper limit value of the Tm value range in the storage means; and
a step of, by the arithmetic means, reading the lower limit value and the upper limit value stored in the storage means and displaying the lower limit value and the upper limit value on display means,
where n is an integer satisfying $2 \leq n$, m is an integer satisfying $2 \leq m \leq n$, N is an integer satisfying $3 \leq n$, i is an integer satisfying $1 \leq i \leq m$, and $m_1$ and $m_2$ are integers satisfying $1 \leq m_1 < m$, $1 \leq m_2 < m$, and $m_1 + m_2 = m$.

[2] The method for designing primers for multiplex PCR according to [1] above, wherein $CV_t = H(CV_0) = \sqrt{2} \times CV_0$ is satisfied.
[3] The method for designing primers for multiplex PCR according to [1] above, wherein $CV_t = H(CV_0) = CV_0$ is satisfied.

According to the present invention, it is possible to provide a method for designing primers for multiplex PCR, in which amplification variations for each region of interest can be suppressed in a plurality of single cells.
The suppression of amplification variations for each region of interest in a plurality of single cells provides stable PCR amplification results across the plurality of single cells, resulting in high-accuracy determination of the number of chromosomes and high-accuracy SNP calling.

## BRIEF DESCRIPTION OF THE DRAWINGS

Fig. 1 is a conceptual diagram illustrating hardware used in the present invention;
Fig. 2 is a flow diagram describing a Tm value range setting step of setting a Tm value range for primer design according to the present invention;
Fig. 3 illustrates histograms for an R1 group and an R2 group, which are obtained as a result of making an attempt N times to PCR amplify m regions of interest out of n regions of interest and to count the number of sequence reads in each region of interest, each histogram having a horizontal axis representing the average Tm value of a pair of primers used to PCR amplify each region of interest and a vertical axis representing the number of regions of interest, in which points "A" to "E" are options for the lower limit value of a Tm value range for primer design, and point "F" is the upper limit value of the Tm value range;

Fig. 4 is a diagram illustrating a first aspect of a method for designing primers for PCR amplifying regions of interest;

Fig. 5 is a diagram illustrating a second aspect of the method for designing primers for PCR amplifying regions of interest; and

Fig. 6 is a diagram illustrating the first aspect of the method for designing primers for PCR amplifying regions of interest.

## DESCRIPTION OF THE PREFERRED EMBODIMENTS

In the present invention, a range indicated using "... to ..." refers to a range including values given before and after "to". For example, "A to B" refers to a range including A and B.

The present invention provides a method for designing primers for multiplex PCR from a single cell, including a Tm value range setting step of setting a Tm value range for primer design.

[Tm Value Range Setting Device]

A device (also referred to as "hardware" or "execution device") that executes a Tm value range setting step according to the present invention will be described with reference to Fig. 1.

In the present invention, the setting of priorities is performed by hardware (device) including arithmetic means (CPU; Central Processing Unit) 11, storage means (memory) 12, auxiliary storage means (storage) 13, input means (keyboard) 14, and display means (monitor) 16. This device may further include auxiliary input means (mouse) 15, output means (printer) 17, and so on.

Each means will be described.

The input means (keyboard) 14 is means for inputting instructions, data, and so on to the device. The auxiliary input means (mouse) 15 is used instead of or together with the input means (keyboard) 14.

The arithmetic means (CPU) 11 is means for performing arithmetic processing.

The storage means (memory) 12 is means for storing results of the arithmetic processing performed by the arithmetic means (CPU) 11 or for storing input from the input means (keyboard) 14.

The auxiliary storage means (storage) 13 is a storage that stores an operating system, a program for determining the necessary number of loci, and so on. A portion of the auxiliary storage means (storage) 13 can also be used for extension of the storage means (memory) 12 (virtual memory).

In the following, the Tm value range setting step and a method for designing primers for PCR amplifying regions of interest will be described.

[Tm Value Range Setting Step]

A description will be given with reference to Fig. 2 and Fig. 3.

The Tm value range setting step is based on the assumption that an attempt to PCR amplify m regions of interest out of n regions of interest and to count the number of sequence reads in each region of interest is made N times to calculate a coefficient of variation for the number of sequence reads in each region of interest, given that an actual value of a coefficient of variation for the number of sequence reads in an i-th region of interest is denoted by $CV_i$ and an average Tm value of a pair of primers used to PCR amplify the i-th region of interest is denoted by $Tm_i$.

In the Tm value range setting step, a Tm value range of a primer is set in the following way.

(1) A target value $CV_0$ of coefficients of variation for the numbers of sequence reads is input from the input means 14 and is stored in the storage means 12 ("input target value ($CV_0$) of coefficient of variation" S11 in Fig. 2).

(2) The number of regions of interest m in the attempt made N times, the actual value $CV_i$ of the coefficient of variation for the number of sequence reads in the i-th region of interest, and the average Tm value $Tm_i$ of the pair of primers used to PCR amplify the i-th region of interest are input and are stored in the storage means 12 ("input primer Tm value (Tmi) and actual value ($CV_i$) of coefficient of variation" S12 in Fig. 2).

(3) The arithmetic means 11 calculates a threshold value $CV_t$ for the coefficients of variation for the numbers of sequence reads as a function of the target value $CV_0$ in accordance with $CV_t = H(CV_0)$, and stores the threshold value $CV_t$ in the storage means 12 ("calculate threshold value $CV_t$) for coefficient of variation" S13 in Fig. 2). The function $H(CV_0)$ of $CV_0$ is not specifically limited, and is preferably given by $H(CV_0) = \sqrt{2} \times CV_0$ or $H(CV_0) = CV_0$, and more preferably given by $H(CV_0) = \sqrt{2} \times CV_0$.

(4) The arithmetic means 11 separates the m regions of interest into an R1 group constituted by $m_1$ regions of interest in which the coefficient of variation $CV_i$ for the number of sequence reads satisfies $CV_i \geq CV_t$ and an R2 group constituted by $m_2$ regions of interest in which the coefficient of variation $CV_i$ for the number of sequence reads satisfies $CV_i < CV_t$, generates respective histograms for the R1 group and the R2 group, each histogram having a horizontal axis representing an average Tm value of a pair of primers used to PCR amplify each region of interest and a vertical axis representing the number of regions of interest, and stores the histograms in the storage means 12 (Fig. 3).

(5) The arithmetic means 11 calculates a value selected in advance from the value at the left end of the histogram for the R1 group ("A" in Fig. 3), the value at the right end of the histogram for the R1 group ("C" in Fig. 3, and the mode of the histogram for the R1 group ("B" in Fig. 3), the value at the left end of the histogram for the R2 group ("E" in Fig. 3), and an intersection ("D" in Fig. 3) of the histogram for the R1 group and the histogram for the R2 group, and stores the calculated value as a lower limit value of the Tm value range in the storage means 12 (""calculate lower limit value of Tm value" S15 in Fig. 2).

(6) The arithmetic means 11 calculates the value at the right end of the R2 group ("F" in Fig. 3), and stores the calculated value as an upper limit value of the Tm value range in the storage means 12 (""calculate upper limit value of Tm value" S16 in Fig. 2).

(7) The arithmetic means 11 displays the Tm value range on the display means 16 or outputs the Tm value range to the output means 17 (""output Tm value range" S17 in Fig. 2).

Note that n is an integer satisfying $2 \leq n$, m is an integer satisfying $2 \leq m \leq n$, N is an integer satisfying $3 \leq n$, i is an integer satisfying $1 \leq i \leq m$, and $m_1$ and $m_2$ are integers satisfying $1 < m_1 < m$, $1 < m_2 < m$, and $m_1 + m_2 = m$.

[Method for Designing Primers for PCR Amplifying Regions of Interest]

In a method for designing primers for multiplex PCR according to the present invention, a method for designing primers for PCR amplifying regions of interest includes the following steps.

<First Aspect of Method for Designing Primers for PCR Amplifying Regions of Interest>

A first aspect of a method for designing primers for PCR amplifying regions of interest includes (a) a target region selection step, (b) a candidate primer base sequence generation step, (c) a local alignment step, (d) a first-stage selection step, (e) a global alignment step, (f) a second-stage selection step, and (g) a primer employment step as below.

(a) A target region selection step of selecting a target region from regions of interest.

(b) A candidate primer base sequence generation step of generating at least one base sequence of a candidate primer for PCR amplifying the target region on the basis of each of base sequences of respective neighboring regions located at two ends of the target region on genomic DNA.

(c) A local alignment step of performing pairwise local alignment, for all combinations for selecting base sequences of two candidate primers from among base sequences of candidate primers generated in the candidate primer base sequence generation step, on two base sequences included in each of the combinations, under a condition in which partial sequences to be compared for the two base sequences include 3'-ends of the two base sequences, to determine local alignment scores.

(d) A first-stage selection step of performing first-stage selection of base sequences of candidate primers for PCR amplifying the target region on the basis of the local alignment scores.

(e) A global alignment step of performing pairwise global alignment, for all combinations for selecting base sequences of two candidate primers from among base sequences of candidate primers selected in the first-stage selection step, on base sequences having a preset sequence length and including 3'-ends of two base sequences included in each of the combinations, to determine global alignment scores.

(f) A second-stage selection step of performing second-stage selection of base sequences of candidate primers for PCR amplifying the target region on the basis of the global alignment scores.

(g) A primer employment step of employing, as base sequences of primers for PCR amplifying the target region, base sequences of candidate primers selected in both the first-stage selection step and the second-stage selection step.

Among the steps (a) to (g), both the steps (c) and (d) and both the steps (e) and (f) may be performed in any order or performed simultaneously. That is, the steps (e) and (f) may be performed after the steps (c) and (d) are performed, or the steps (c) and (d) may be performed after the steps (e) and (f) are performed. Alternatively, the steps (c) and (d) and the steps (e) and (f) may be performed in parallel.

If the steps (c) and (d) are performed after the steps (e) and (f) are performed, the steps (e) and (c) are preferably replaced with steps (e') and (c') below, respectively.

(e') A global alignment step of performing pairwise global alignment, for all combinations for selecting base sequences of two candidate primers from among base sequences of candidate primers generated in the candidate primer base sequence generation step, on base sequences having a preset sequence length and including 3'-ends of two base sequences included in each of the combinations, to determine global alignment scores.

(c') A local alignment step of performing pairwise local alignment, for all combinations for selecting base sequences of two candidate primers from among base sequences of candidate primers selected in the second-stage selection step, on two base sequences included in each of the combinations, under a condition in which partial sequences to be compared for the two base sequences include 3'-ends of the two base sequences, to determine local alignment scores.

Further, if the steps (c) and (d) and the steps (e) and (f) are performed in parallel, the step (e) is preferably replaced with step (e') below.

(e') A global alignment step of performing pairwise global alignment, for all combinations for selecting base sequences of two candidate primers from among base sequences of candidate primers generated in the candidate primer base sequence generation step, on base sequences having a preset sequence length and including 3'-ends of two base sequences included in each of the combinations, to determine global alignment scores.

<Second Aspect of Method for Designing Primers for PCR Amplifying Regions of Interest>

A second aspect of the method for designing primers for PCR amplifying regions of interest includes the following: ($a_1$) a first step of target region selection, ($b_1$) a first step of candidate primer base sequence generation, ($c_1$) a first step of local alignment, ($d_1$) a first step of first-stage selection, ($e_1$) a first step of global alignment, ($f_1$) a first step of second-stage selection, ($g_1$) a first step of primer employment, ($a_2$) a second step of target region selection, ($b_2$) a second step of candidate primer base sequence generation, ($c_2$) a second step of local alignment, ($d_2$) a second step of first-stage selection, ($e_2$) a second step of global alignment, ($f_2$) a second step of second-stage selection, and ($g_2$) a second step of primer employment as below.

($a_1$) A first step of target region selection for selecting a first target region from regions of interest.

($b_1$) A first step of candidate primer base sequence generation for generating at least one base sequence of a candidate primer for PCR amplifying the first target region on the basis of each of base sequences of respective neighboring regions located at two ends of the first target region on genomic DNA.

($c_1$) A first step of local alignment for performing pairwise local alignment, for all combinations for selecting base sequences of two candidate primers from among base sequences of candidate primers generated in the first step of candidate primer base sequence generation, on two base sequences included in each of the combinations, under a condition in which partial sequences to be compared for the two base sequences include 3'-ends of the two base sequences, to determine local alignment scores.

($d_1$) A first step of first-stage selection for performing first-stage selection of base sequences of candidate primers for PCR amplifying the first target region on the basis of the local alignment scores.

($e_1$) A first step of global alignment for performing pairwise global alignment, for all combinations for selecting base sequences of two candidate primers from among base sequences of candidate primers selected in the first step of first-stage selection, on base sequences having a preset sequence length and including 3'-ends of two base sequences included in each of the combinations, to determine global alignment scores.

($f_1$) A first step of second-stage selection for performing second-stage selection of base sequences of candidate primers for PCR amplifying the first target region on the basis of the global alignment scores.

($g_1$) A first step of primer employment for employing, as base sequences of primers for PCR amplifying the first target region, base sequences of candidate primers selected in both the first step of first-stage selection and the first step of second-stage selection.

($a_2$) A second step of target region selection for selecting a second target region different from the first target region from regions of interest.

($b_2$) A second step of candidate primer base sequence generation for generating at least one base sequence of a candidate primer for PCR amplifying the second target region on the basis of each of base sequences of respective neighboring regions located at two ends of the second target region on genomic DNA.

($c_2$) A second step of local alignment for performing pairwise local alignment, for all combinations for selecting base sequences of two candidate primers and all combinations for selecting a base sequence of one candidate primer and a base sequence of one primer that has already been employed from among base sequences of candidate primers generated in the second step of candidate primer base sequence generation and from among base sequences of primers that have already been employed, on two base sequences included in each of the combinations, under a condition in which partial sequences to be compared for the two base sequences include 3'-ends of the two base sequences, to determine local alignment scores.

($d_2$) A second step of first-stage selection for performing first-stage selection of base sequences of candidate primers for PCR amplifying the second target region on the basis of the local alignment scores.

($e_2$) A second step of global alignment for performing pairwise global alignment, for all combinations for selecting base sequences of two candidate primers and all combinations for selecting a base sequence of one candidate primer and a base sequence of one primer that has already been employed from among base sequences of candidate primers selected in the second step of first-stage selection and from among base sequences of primers that have already been employed, on base sequences having a preset sequence length and including 3'-ends of two base sequences included in each of the combinations, to determine global alignment scores.

($f_2$) A second step of second-stage selection for performing second-stage selection of base sequences of candidate primers for PCR amplifying the second target region on the basis of the global alignment scores.

($g_2$) A second step of primer employment for employing, as base sequences of primers for PCR amplifying the second target region, base sequences of candidate primers selected in both the second step of first-stage selection and the second step of second-stage selection.

Among the steps ($a_1$) to ($g_1$), both the steps ($c_1$) and ($d_1$) and both the steps ($e_1$) and ($f_1$) may be performed in any order or performed simultaneously. That is, the steps ($e_1$) and ($f_1$) may be performed after the steps ($c_1$) and ($d_1$) are performed, or the steps ($c_1$) and ($d_1$) may be performed after the steps ($e_1$) and ($f_1$) are performed. Alternatively, the steps ($c_1$) and ($d_1$) and the steps ($e_1$) and ($f_1$) may be performed in parallel.

If the steps ($c_1$) and ($d_1$) are performed after the steps ($e_1$) and ($f_1$) are performed, the steps ($e_1$) and ($c_1$) are preferably replaced with steps ($e_1$') and ($c_1$') below, respectively.

($e_1$') A first step of global alignment for performing pairwise global alignment, for all combinations for selecting base sequences of two candidate primers from among base sequences of candidate primers generated in the first step of candidate primer base sequence generation, on base sequences having a preset sequence length and including 3'-ends of two base sequences included in each of the combinations, to determine global alignment scores.

($c_1$') A first step of local alignment for performing pairwise local alignment, for all combinations for selecting base sequences of two candidate primers from among base sequences of candidate primers selected in the first step of second-stage selection, on two base sequences included in each of the combinations, under a condition in which partial sequences to be compared for the two base sequences include 3'-ends of the two base sequences, to determine local alignment scores.

Further, if the steps ($c_1$) and ($d_1$) and the steps ($e_1$) and ($f_1$) are performed in parallel, the step ($e_1$) is preferably replaced with step ($e_1$') below.

($e_1$') A first step of global alignment for performing pairwise global alignment, for all combinations for selecting base sequences of two candidate primers from among base sequences of candidate primers generated in the first step of candidate primer base sequence generation, on base sequences having a preset sequence length and including 3'-ends of two base sequences included in each of the combinations, to determine global alignment scores.

Among the steps ($a_2$) to ($g_2$), both the steps ($c_2$) and ($d_2$) and both the steps ($e_2$) and ($f_2$) may be performed in any order or performed simultaneously. That is, the steps ($e_2$) and ($f_2$) may be performed after the steps ($c_2$) and ($d_2$) are performed, or the steps ($c_2$) and ($d_2$) may be performed after the steps ($e_2$) and ($f_2$) are performed. Alternatively, the steps ($c_2$) and ($d_2$) and the steps ($e_2$) and ($f_2$) may be performed in parallel.

If the steps ($c_2$) and ($d_2$) are performed after the steps ($e_2$) and ($f_2$) are performed, the steps ($e_2$) and ($c_2$) are preferably replaced with steps ($e_2$') and ($c_2$') below, respectively.

($e_2$') A second step of global alignment for performing pairwise global alignment, for all combinations for selecting base sequences of two candidate primers and all combinations for selecting a base sequence of one candidate primer and a base sequence of one primer that has already been employed from among base sequences of candidate primers generated in the second step of candidate primer base sequence generation and from among base sequences of primers that have already been employed, on base sequences having a preset sequence length and including 3'-ends of two base sequences included in each of the combinations, to determine global alignment scores.

($c_2$') A second step of local alignment for performing pairwise local alignment, for all combinations for selecting base sequences of two candidate primers and all combinations for selecting a base sequence of one candidate primer and a base sequence of one primer that has already been employed from among base sequences of candidate primers selected in the second step of second-stage selection and from among base sequences of primers that have already been employed, on two base sequences included in each of the combinations, under a condition in which partial sequences to be compared for the two base sequences include 3'-ends of the two base sequences, to determine local alignment scores.

Further, if the steps ($c_2$) and ($d_2$) and the steps ($e_2$) and ($f_2$) are performed in parallel, the step ($e_2$) is preferably replaced with step ($e_2$') below.

($e_2$') A second step of global alignment for performing pairwise global alignment, for all combinations for selecting base sequences of two candidate primers and all combinations for selecting a base sequence of one candidate primer and a base sequence of one primer that has already been employed from among base sequences of candidate primers generated in the second step of candidate primer base sequence generation and from among base sequences of primers that have already been employed, on base sequences having a preset sequence length and including 3'-ends of two base sequences included in each of the combinations, to determine global alignment scores.

Further, when the at least one region of interest includes three or more regions of interest and when base sequences of primers for PCR amplifying third and subsequent target regions that have not yet been selected from the three or more regions of interest are employed, the steps (a2) to (g2) are repeated for each of the third and subsequent target regions.

<Third Aspect of Method for Designing Primers for PCR Amplifying Regions of Interest>

A third aspect of the method for designing primers for PCR amplifying regions of interest includes the following: (a-0) a plurality-of-target-region selection step, (b-0) a plurality-of-candidate-primer-base-sequence generation step, (c-1) a first local alignment step, (d-1) a first first-stage selection step, (e-1) a first global alignment step, (f-1) a first second-stage selection step, (g-1) a first primer employment step, (c-2) a second local alignment step, (d-2) a second first-stage selection step, (e-2) a second global alignment step, (f-2) a second second-stage selection step, and (g-2) a second primer employment step as below.

(a-0) A plurality-of-target-region selection step of selecting a plurality of target regions from regions of interest.

(b-0) A plurality-of-candidate-primer-base-sequence generation step of generating at least one base sequence of a candidate primer for PCR amplifying each of the plurality of target regions on the basis of each of base sequences of respective neighboring regions located at two ends of each of the plurality of target regions on genomic DNA.

(c-1) A first local alignment step of setting, as a first target region, one of the plurality of target regions selected in the plurality-of-target-region selection step, and performing pairwise local alignment, for all combinations for selecting base sequences of two candidate primers from a base sequence of a candidate primer for PCR amplifying the first target region among base sequences of candidate primers generated in the plurality-of-candidate-primer-base-sequence generation step, on two base sequences included in each of the combinations, under a condition in which partial sequences to be compared for the two base sequences include 3'-ends of the two base sequences, to determine local alignment scores.

(d-1) A first first-stage selection step of performing first-stage selection of base sequences of candidate primers for PCR amplifying the first target region on the basis of the local alignment scores.

(e-1) A first global alignment step of performing pairwise global alignment, for all combinations for selecting base sequences of two candidate primers from among base sequences of candidate primers selected in the first first-stage selection step, on base sequences having a preset sequence length and including 3'-ends of two base sequences included in each of the combinations, to determine global alignment scores.

(f-1) A first second-stage selection step of performing second-stage selection of base sequences of candidate primers for PCR amplifying the first target region on the basis of the global alignment scores.

(g-1) A first primer employment step of employing, as base sequences of primers for PCR amplifying the first target region, base sequences of candidate primers selected in both the first first-stage selection step and the first second-stage selection step.

(c-2) A second local alignment step of setting, as a second target region, one of the plurality of target regions selected in the plurality-of-target-region selection step, except for the first target region, and performing pairwise local alignment, for all combinations for selecting base sequences of two candidate primers and all combinations for selecting a base sequence of one candidate primer and a base sequence of one primer that has already been employed from among base sequences of candidate primers for PCR amplifying the second target region and from among base sequences of primers that have already been employed among base sequences of candidate primers generated in the plurality-of-candidate-primer-base-sequence generation step, on two base sequences included in each of the combinations, under a condition in which partial sequences to be compared for the two base sequences include 3'-ends of the two base sequences, to determine local alignment scores.

(d-2) A second first-stage selection step of performing first-stage selection of base sequences of candidate primers for PCR amplifying the second target region on the basis of the local alignment scores.

(e-2) A second global alignment step of performing pairwise global alignment, for all combinations for selecting base sequences of two candidate primers and all combinations for selecting a base sequence of one candidate primer and a base sequence of one primer that has already been employed from among base sequences of candidate primers selected in the second first-stage selection step and from among base sequences of primers that have already been employed, on base sequences having a preset sequence length and including 3'-ends of two base sequences included in each of the combinations, to determine global alignment scores.

(f-2) A second second-stage selection step of performing second-stage selection of base sequences of candidate primers for PCR amplifying the second target region on the basis of the global alignment scores.

(g-2) A second primer employment step of employing, as base sequences of primers for PCR amplifying the second target region, base sequences of candidate primers selected in both the second first-stage selection step and the second second-stage selection step.

Among the steps (c-1) to (g-1), both the steps (c-1) and (d-1) and both the steps (e-1) and (f-1) may be performed in any order or performed simultaneously. That is, the steps (e-1) and (f-1) may be performed after the steps (c-1) and (d-1) are performed, or the steps (c-1) and (d-1) may be performed after the steps (e-1) and (f-1) are performed. Alternatively, the steps (c-1) and (d-1) and the steps (e-1) and (f-1) may be performed in parallel.

If the steps (c-1) and (d-1) are performed after the steps (e-1) and (f-1) are performed, the steps (e-1) and (c-1) are preferably replaced with steps (e'-1) and (c'-1) below, respectively.

(e'-1) A first global alignment step of setting, as a first target region, one of the plurality of target regions selected in the plurality-of-target-region selection step, and performing pairwise global alignment, for all combinations for selecting base sequences of two candidate primers from a base sequence of a candidate primer for PCR amplifying the first target region among base sequences of candidate primers generated in the plurality-of-candidate-primer-base-sequence generation step, on base sequences having a preset sequence length and including 3'-ends of two base sequences included in each of the combinations, to determine global alignment scores.

(c'-1) A first local alignment step of performing pairwise local alignment, for all combinations for selecting base sequences of two candidate primers from among base sequences of candidate primers selected in the first second-stage selection step, on two base sequences included in each of the combinations, under a condition in which partial sequences to be compared for the two base sequences include 3'-ends of the two base sequences, to determine local alignment scores.

Further, if the steps (c-1) and (d-1) and the steps (e-1) and (f-1) are performed in parallel, the step (e-1) is preferably replaced with step (e'-1) below.

(e'-1) A first global alignment step of setting, as a first target region, one of the plurality of target regions selected in the plurality-of-target-region selection step, and performing pairwise global alignment, for all combinations for selecting base sequences of two candidate primers from a base sequence of a candidate primer for PCR amplifying the first target region among base sequences of candidate primers generated in the plurality-of-candidate-primer-base-sequence generation step, on base sequences having a preset sequence length and including 3'-ends of two base sequences included in each of the combinations, to determine global alignment scores.

Among the steps (c-2) to (g-2), both the steps (c-2) and (d-2) and both the steps (e-2) and (f-2) may be performed in any order or performed simultaneously. That is, the steps (e-2) and (f-2) may be performed after the steps (c-2) and (d-2) are performed, or the steps (c-2) and (d-2) may be performed after the steps (e-2) and (f-2) are performed. Alternatively, the steps (c-1) and (d-1) and the steps (e-1) and (f-1) may be performed in parallel.

If the steps (c-2) and (d-2) are performed after the steps (e-2) and (f-2) are performed, the steps (e-2) and (c-2) are preferably replaced with steps (e'-2) and (c'-2) below, respectively.

(e'-2) A second global alignment step of setting, as a second target region, one of the plurality of target regions selected in the plurality-of-target-region selection step, except for the first target region, and performing pairwise global alignment, for all combinations for selecting base sequences of two candidate primers and all combinations for selecting a base sequence of one candidate primer and a base sequence of one primer that has already been employed from among base sequences of candidate primers for PCR amplifying the second target region and from among base sequences of primers that have already been employed among base sequences of candidate primers generated in the plurality-of-candidate-primer-base-sequence generation step, on base sequences having a preset sequence length and including 3'-ends of two base sequences included in each of the combinations, to determine global alignment scores.

(c'-2) A second local alignment step of performing pairwise local alignment, for all combinations for selecting base sequences of two candidate primers and all combinations for selecting a base sequence of one candidate primer and a base sequence of one primer that has already been employed from among base sequences of candidate primers selected in the second second-stage selection step and from among base sequences of primers that have already been employed, on two base sequences included in each of the combinations, under a condition in which partial sequences to be compared for the two base sequences include 3'-ends of the two base sequences, to determine local alignment scores.

Further, if the steps (c-2) and (d-2) and the steps (e-2) and (f-2) are performed in parallel, the step (e-2) is preferably replaced with step (e'-2) below.

(e'-2) A second global alignment step of setting, as a second target region, one of the plurality of target regions selected in the plurality-of-target-region selection step, except for the first target region, and performing pairwise global alignment for all combinations for selecting base sequences of two candidate primers and all combinations for selecting a base sequence of one candidate primer and a base sequence of one primer that has already been employed from among base sequences of candidate primers for PCR amplifying the second target region and from among base sequences of primers that have already been employed among base sequences of candidate primers generated in the plurality-of-candidate-primer-base-sequence generation step, on base sequences having a preset sequence length and including 3'-ends of two base sequences included in each of the combinations, to determine global alignment scores.

Further, when the at least one region of interest includes three or more regions of interest, when three or more target regions are selected in the plurality-of-target-region selection step, when base sequences of candidate primers for PCR amplifying each of the three or more target regions are generated in the plurality-of-candidate-primer-base-sequence generation step, and when one of the plurality of target regions selected in the plurality-of-target-region selection step, except for the first and second target regions, is set as a third target region and base sequences of primers for PCR amplifying the third and subsequent target regions are employed, the steps from the second local alignment step to the second primer employment step are repeated for the third and subsequent target regions.

<Description of Steps>

The steps in the first to third aspects of the method for designing primers for PCR amplifying regions of interest will be described with reference to Fig. 4 to Fig. 6, as appropriate.

<<Target Region Selection Step>>

As used herein, target region selection step S101 (Fig. 4), first step of target region selection S201 and second step of target region selection S211 (Fig. 5), and plurality-of-target-region selection step S301 (Fig. 6) are collectively referred to sometimes simply as "target region selection step".

(First aspect: target region selection step S101)

In Fig. 4, this step is represented as "target region selection".

In the first aspect, the target region selection step (a) is a step of selecting a target region from regions of interest. The method for selection is not specifically limited, and, for example, when the regions of interest are assigned priorities for primer design, target regions in which primers are designed are selected from among the regions of interest in order of priority.

(Second aspect: first step of target region selection S201 and second step of target region selection S211)

In Fig. 5, these steps are represented as "target region selection: first" and "target region selection: second".

In the second aspect, the first step of target region selection ($a_1$) is a step of selecting a first target region from regions of interest, and the second step of target region selection ($a_2$) is a step of selecting a second target region from regions of interest that are yet to be selected as target regions. The method for selection is not specifically limited, and, for example, when the regions of interest are assigned priorities for primer design, target regions in which primers are designed are selected from among the regions of interest in order of priority.

(Third aspect: plurality-of-target-region selection step S301)

In Fig. 6, this step is represented as "plurality-of-target-region selection".

In the third aspect, the plurality-of-target-region selection step (a-0) is a step of selecting a plurality of target regions from regions of interest. The method for selection is not specifically limited, and, for example, when the regions of interest are assigned priorities for primer design, a plurality of target regions in which primers are designed are selected from among the regions of interest in order of priority.

<<Candidate Primer Base Sequence Generation Step>>

As used herein, candidate primer base sequence generation step S102 (Fig. 4), first step of candidate primer base sequence generation S202 and second step of candidate primer base sequence generation S212 (Fig. 5), and plurality-of-candidate-primer-base-sequence generation step S302 (Fig. 6) are collectively referred to sometimes simply as "candidate primer base sequence generation step".

(First aspect: candidate primer base sequence generation step S102)

In Fig. 4, this step is represented as "candidate primer base sequence generation".
In the first aspect, the candidate primer base sequence generation step (b) is a step of generating at least one base sequence of a candidate primer for PCR amplifying a target region on the basis of each of base sequences of respective neighboring regions located at two ends of the target region on genomic DNA.

(Second aspect: first step of candidate primer base sequence generation S202 and second step of candidate primer base sequence generation S212)

In Fig. 5, these steps are represented as "candidate primer base sequence generation: first" and "candidate primer base sequence generation: second".
In the second aspect, the first step of candidate primer base sequence generation ($b_1$) is a step of generating at least one base sequence of a candidate primer for PCR amplifying a first target region on the basis of each of base sequences of respective neighboring regions located at two ends of the first target region on genomic DNA, and the second step of candidate primer base sequence generation ($b_2$) is a step of generating at least one base sequence of a candidate primer for PCR amplifying a second target region on the basis of each of base sequences of respective neighboring regions located at two ends of the second target region on genomic DNA.
In the second aspect, the generation of a base sequence of a candidate primer, the selection of a candidate primer, and the employment of a primer are performed for one target region, and similar steps are repeated for the next target region.

(Third aspect: plurality-of-candidate-primer-base-sequence generation step S302)

In Fig. 6, this step is represented as "plurality-of-candidate-primer-base-sequence generation".
In the third aspect, the plurality-of-candidate-primer-base-sequence generation step (b-0) generates at least one base sequence of a candidate primer for PCR amplifying each of a plurality of target regions on the basis of each of base sequences of respective neighboring regions located at two ends of each of the plurality of target regions on genomic DNA.
In the third aspect, base sequences of candidate primers are generated for all the plurality of target regions, and selection and employment are repeated in the subsequent steps.

(Neighboring region)

Respective neighboring regions located at two ends of a target region are collectively referred to as regions outside the 5'-end of the target region and regions outside the 3'-end of the target region. The area inside the target region is not included in the neighboring regions.
The length of a neighboring region is not specifically limited, and is preferably less than or equal to a length that allows extension of a neighboring region by PCR, and more preferably less than or equal to the upper limit of the length of the DNA fragment to be amplified. In particular, the length of a neighboring region is preferably a length that facilitates application of concentration selection and/or sequence reading. The length of a neighboring region may be changed as appropriate in accordance with the type or the like of enzyme (DNA polymerase) to be used in PCR. The specific length of a neighboring region is preferably about 20 to 500 bases, more preferably about 20 to 300 bases, even more preferably about 20 to 200 bases, and still more preferably about 50 to 200 bases.

(Primer design parameter)

In addition, to generate a base sequence of a candidate primer, careful attention is required to the same points as those in a common method for designing primers, such as primer length, GC content (corresponding to the total mole percentage of guanine (G) and cytosine (C) in all nucleic acid bases), melting temperature (temperature at which 50% of double-stranded DNA is dissociated into single-stranded DNA, referred to sometimes as "Tm value", from Melting Temperature, in "°C"), and sequence deviation.

• Primer Length

The primer length (number of nucleotides) is not specifically limited, and is preferably 15-mer to 45-mer, more preferably 20-mer to 45-mer, and even more preferably 20-mer to 30-mer. A primer length in this range facilitates the designing of a primer excellent in specificity and amplification efficiency.

• Primer GC Content

The primer GC content is not specifically limited, and is preferably 40 mol% to 60 mol%, and more preferably 45 mol% to 55 mol%. A GC content in this range is less likely to cause a problem of a reduction in specificity and amplification efficiency due to a high-order structure.

• Primer Tm Value

The primer Tm value is not specifically limited, and is preferably in a range of 50°C to 65°C, and more preferably in a range of 55°C to 65°C.
In a primer pair and a primer set, the difference between the Tm values of primers is set to preferably 5°C or less, and more preferably 3°C or less.
The Tm value can be calculated using software such as OLIGO Primer Analysis Software (manufactured by Molecular Biology Insights Inc.) or Primer3 (http://www-genome.wi.mit.edu/ftp/distribution/software/).
Alternatively, the Tm value can be calculated in accordance with the formula below based on the numbers of A's, T's, G's, and C's (represented as nA, nT, nG, and nC, respectively) in a base sequence of a primer.

$$\text{Tm value } (^\circ\text{C}) = 2(\text{nA} + \text{nT}) + 4(\text{nC} + \text{nG})$$

The method for calculating the Tm value is not limited to those described above, and the Tm value can be calculated using any of various well-known methods.
Note that in the method for designing primers for multiplex PCR according to the present invention, after a range of Tm values is set in the "Tm value range setting step of setting a Tm value range for primer design" described above, the range of Tm values is used.

• Base Deviation of Primer

A base sequence of a candidate primer is preferably a sequence having entirely no deviation of bases. For example, it is desirable to avoid a partially GC-rich sequence and a partially AT-rich sequence.
It is also desirable to avoid consecutive T's and/or C's (polypyrimidine) and consecutive A's and/or G's (polypurine).

• 3'-end of Primer

For the 3'-end base sequence, furthermore, it is preferable to avoid a GC-rich sequence or an AT-rich sequence. The base at the 3'-end is preferably, but is not limited to, G or C.

<<Specificity Check Step>>

A specificity check step may be performed (not illustrated) to evaluate the specificity of a base sequence of a candidate primer on the basis of the sequence complementarity of a base sequence of each candidate primer, which is generated in the "candidate primer base sequence generation step", to chromosomal DNA.
A specificity check may be performed in the following manner. Local alignment is performed between a base sequence of chromosomal DNA and a base sequence of a candidate primer, and it can be evaluated that the base sequence of the candidate primer has low complementarity to the genomic DNA and has high specificity when the local alignment score is less than a preset value. It is desirable to perform local alignment also on complementary strands of the chromosomal DNA. This is because whereas a primer is single-stranded DNA, chromosomal DNA is double-stranded. Alternatively, instead of a base sequence of a candidate primer, a base sequence complementary thereto may be used.

In addition, homology search may be performed against a genomic DNA base sequence database by using a base sequence of a candidate primer as a query sequence. Examples of a homology search tool include BLAST (Basic Local Alignment Search Tool) (Altschul, S. A., four others, "Basic Local Alignment Search Tool", Journal of Molecular Biology, October 1990, Vol. 215, pp. 403-410) and FASTA (Pearson, W. R., one other, "Improved tools for biological sequence comparison", Proceedings of the National Academy of Sciences of the United States of America, the National Academy of Sciences of the United States of America, April 1988, Vol. 85, pp. 2444-2448). As a result of homology search, local alignment can be obtained.

Threshold values for scores and local alignment scores are not specifically limited and may be set as appropriate in accordance with the length of a base sequence of a candidate primer and/or PCR conditions or the like. When a homology search tool is used, specified values for the homology search tool may be used.

For example, as the score, match (complementary base) = +1, mismatch (non-complementary base) = -1, and indel (insertion and/or deletion) = -3 may be employed, and the threshold value may be set to +15.

If a base sequence of a candidate primer has complementarity to a base sequence at an unexpected position on chromosomal DNA and has low specificity, an artifact, rather than a target region, may be amplified in PCR performed using a primer of the base sequence, and the artifact is thus removed.

<<Local Alignment Step>>

As used herein, local alignment step S103 (Fig. 4), first step of local alignment S203 and second step of local alignment S213 (Fig. 5), and first local alignment step S303 and second local alignment step S313 (Fig. 6) are collectively referred to sometimes simply as "local alignment step".

(First aspect: local alignment step S103)

In Fig. 4, this step is represented as "local alignment".

In the first aspect, the local alignment step (c) is a step of performing pairwise local alignment, for all combinations for selecting base sequences of two candidate primers from among base sequences of candidate primers generated in the candidate primer base sequence generation step, on two base sequences included in each of the combinations, under a condition in which partial sequences to be compared for the two base sequences include 3'-ends of the two base sequences, to determine local alignment scores.

(Second aspect: first step of local alignment S203 and second step of local alignment S213)

In Fig. 5, these steps are represented as "local alignment: first" and "local alignment: second".

In the second aspect, the first step of local alignment ($c_1$) is a step of performing pairwise local alignment, for all combinations for selecting base sequences of two candidate primers from among base sequences of candidate primers generated in the first step of candidate primer base sequence generation, on two base sequences included in each of the combinations, under a condition in which partial sequences to be compared for the two base sequences include 3'-ends of the two base sequences, to determine local alignment scores, and the second step of local alignment ($c_2$) is a step of performing pairwise local alignment, for all combinations for selecting base sequences of two candidate primers and all combinations for selecting a base sequence of one candidate primer and a base sequence of one primer that has already been employed from among base sequences of candidate primers generated in the second step of candidate primer base sequence generation and from among base sequences of primers that have already been employed, on two base sequences included in each of the combinations, under a condition in which partial sequences to be compared for the two base sequences include 3'-ends of the two base sequences, to determine local alignment scores.

(Third aspect: first local alignment step S303 and second local alignment step S313)

In Fig. 6, these steps are represented as "first local alignment" and "second local alignment".

In the third aspect, the first local alignment step (c-1) is a step of performing pairwise local alignment, for all combinations for selecting base sequences of two candidate primers from among base sequences of candidate primers for PCR amplifying the first target region among base sequences of candidate primers generated in the plurality-of-candidate-primer-base-sequence generation step, on two base sequences included in each of the combinations, under a condition in which partial sequences to be compared for the two base sequences include 3'-ends of the two base sequences, to determine local alignment scores, and the second local alignment step (c-2) is a step of performing pairwise local alignment, for all combinations for selecting base sequences of two candidate primers and all combinations for selecting a base sequence of one candidate primer and a base sequence of one primer that has already been employed from among base sequence of candidate primer for PCR amplifying the second target region among base sequences of candidate primers generated in the plurality-of-candidate-primer-base-sequence generation step and from among base sequences of primers that have already been employed, on two base sequences included in each of the combinations, under a condition in which partial sequences to be compared for the two base sequences include 3'-ends of the two base sequences, to determine local alignment scores.

(Method for local alignment)

A combination of base sequences to be subjected to local alignment may be a combination selected with allowed overlap or a combination selected without allowed overlap. However, if the probability of primer dimer formation between primers having the same base sequence has not yet been evaluated, it is preferable to use a combination selected with allowed overlap.

The total number of combinations is given by "${}_pH_2 = {}_{p+1}C_2 = (p + 1)!/2(p - 1)!$" when combinations are selected with allowed overlap, and is given by "${}_pC_2 = p(p - 1)/2$" when combinations are selected without allowed overlap, where p denotes the total number of base sequences to be subjected to local alignment.

Local alignment is alignment to be performed on partial sequences and allows local examination of high complementarity fragments.

In the present invention, however, unlike typical local alignment performed on base sequences, local alignment is performed under the condition that "partial sequences to be compared include the 3'-ends of the base sequences", so that partial sequences to be compared include the 3'-ends of both the base sequences.

In the present invention, furthermore, in a preferred aspect, local alignment is performed under the condition that "partial sequences to be compared include the 3'-ends of the base sequences", that is, the condition that "partial sequences to be compared take into account only alignment that starts at the 3'-end of one of the sequences and ends at the 3'-end of the other sequence", so that partial sequences to be compared include the 3'-ends of both the base sequences.

Note that in local alignment, a gap may be inserted. The gap refers to an insertion and/or deletion (indel) of a base.

In local alignment, furthermore, a match is determined when bases in a base sequence pair are complementary to each other, and a mismatch is determined when bases in a base sequence pair are not complementary to each other.

Alignment is performed such that a score is set for each of a match, a mismatch, and an indel and the total score is maximum. The scores may be set as appropriate. For example, scores may be set as in Table 1 below. In Table 1, "-" indicates a gap (insertion and/or deletion (indel)).

Table 1

| | A | T | G | C | - |
|---|---|---|---|---|---|
| A | -1 | +1 | -1 | -1 | -1 |
| T | +1 | -1 | -1 | -1 | 1 |
| G | -1 | -1 | -1 | +1 | -1 |
| C | -1 | -1 | +1 | -1 | -1 |
| - | -1 | -1 | -1 | -1 | |

"-":gap(indel)

For example, consideration is given to local alignment of base sequences with SEQ ID NOs: 1 and 2 given in Table 2 below. Here, scores are assumed to be given in Table 1.

Table 2

| | Base sequence (5' → 3') |
|---|---|
| SEQ ID NO: 1 | CTTCGATGCGGACCTTCTGG |
| SEQ ID NO: 2 | TCTCCCACATCCGGCTATGG |

A dot matrix given in Table 3 is generated from the base sequences with SEQ ID NOs: 1 and 2. Specifically, the base sequence with SEQ ID NO: 1 is arranged from left to right in a 5' to 3' direction, and the base sequence with SEQ ID NO: 2 is arranged from bottom to top in a 5' to 3' direction, with grids of complementary bases filled with "●" to obtain a dot matrix given in Table 3.

Table 3

| | | SEQ ID NO: 1→ | | | | | | | | | | | | | | | | | | | |
|---|---|---|---|---|---|---|---|---|---|---|---|---|---|---|---|---|---|---|---|---|---|
| | | C | T | T | C | G | A | T | G | C | G | G | A | C | C | T | T | C | T | G | G |
| SEQ ID NO: 2→ | G | ● | | | ● | | | | | ● | | | | ● | ● | | | ● | | | |
| | G | ● | | | ● | | | | | ● | | | | ● | ● | | | ● | | | |
| | T | | | | | | ● | | | | | | ● | | | | | | | | |
| | A | | ● | ● | | | | ● | | | | | | | | ● | ● | | ● | | |
| | T | | | | | | ● | | | | | | ● | | | | | | | | |
| | C | | | | | ● | | | ● | | ● | ● | | | | | | | | ● | ● |
| | G | ● | | | ● | | | | | ● | | | | ● | ● | | | ● | | | |
| | G | ● | | | ● | | | | | ● | | | | ● | ● | | | ● | | | |
| | C | | | | | ● | | | ● | | ● | ● | | | | | | | | ● | ● |
| | C | | | | | ● | | | ● | | ● | ● | | | | | | | | ● | ● |
| | T | | | | | | ● | | | | | | ● | | | | | | | | |
| | A | | ● | ● | | | | ● | | | | | | | | ● | ● | | ● | | |
| | C | | | | | ● | | | ● | | ● | ● | | | | | | | | ● | ● |
| | A | | ● | ● | | | | ● | | | | | | | | ● | ● | | ● | | |
| | C | | | | | ● | | | ● | | ● | ● | | | | | | | | ● | ● |
| | C | | | | | ● | | | ● | | ● | ● | | | | | | | | ● | ● |
| | C | | | | | ● | | | ● | | ● | ● | | | | | | | | ● | ● |
| | T | | | | | | ● | | | | | | ● | | | | | | | | |
| | C | | | | | ● | | | ● | | ● | ● | | | | | | | | ● | ● |
| | T | | | | | | ● | | | | | | ● | | | | | | | | |

The dot matrix given in Table 3 yields alignment of partial sequences (pairwise alignment) as given in Table 4 below (see a portion indicated by the diagonal line in Table 3). In Table 4, a match is denoted by "|" and a mismatch is denoted by ":".

Table 4

| | | | | | | | | |
|---|---|---|---|---|---|---|---|---|
| Partial sequence from SEQ ID NO: 1 | 5'- | T | T | C | T | G | G | -3' |
| | | : | : | : | \| | : | \| | |
| Partial sequence from SEQ ID NO: 2 | 3'- | G | G | T | A | T | C | -5' |

This (pairwise) alignment includes two matches, four mismatches, and no indel (gap).

Thus, the local alignment score based on this (pairwise) alignment is given by $(+1) \times 2 + (-1) \times 4 + (-1) \times 0 = -2$.

Note that the alignment (pairwise alignment) may be obtained using, instead of the dot matrix method exemplified herein, the dynamic programming method, the word method, or any of various other methods.

<<First-Stage Selection Step>>

As used herein, first-stage selection step S104 (Fig. 4), first step of first-stage selection S204 and second step of first-stage selection S214 (Fig. 5), and first first-stage selection step S304 and second first-stage selection step S314 (Fig. 6) are collectively referred to sometimes simply as "first-stage selection step".

(First aspect: first-stage selection step S104)

In Fig. 4, this step is represented as "first-stage selection".

In the first aspect, the first-stage selection step (d) is a step of performing first-stage selection of base sequences of candidate primers for PCR amplifying the target region on the basis of the local alignment scores.

(Second aspect: first step of first-stage selection S204 and second step of first-stage selection S214)

In Fig. 5, these steps are represented as "first-stage selection: first" and "first-stage selection: second".

In the second aspect, the first step of first-stage selection ($d_1$) is a step of performing first-stage selection of base sequences of candidate primers for PCR amplifying the first target region on the basis of the local alignment scores, and the second step of first-stage selection ($d_2$) is a step of performing first-stage selection of base sequences of candidate primers for PCR amplifying the second target region on the basis of the local alignment scores.

(Third aspect: first first-stage selection step S304 and second first-stage selection step S314)

In Fig. 6, these steps are represented as "first first-stage selection" and "second first-stage selection".

In the third aspect, the first first-stage selection step (d-1) is a step of performing first-stage selection of base sequences of candidate primers for PCR amplifying the first target region on the basis of the local alignment scores, and the second first-stage selection step (d-2) is a step of performing first-stage selection of base sequences of candidate primers for PCR amplifying the second target region on the basis of the local alignment scores.

(Method for first-stage selection)

A threshold value for local alignment scores (referred to also as "first threshold value") is set in advance.

If a local alignment score is less than the first threshold value, the combination of two base sequences is determined to have low probability of dimer formation, and then the subsequent step is performed.

On the other hand, if a local alignment score is not less than the first threshold value, the combination of two base sequences is determined to have high probability of primer dimer formation, and no further steps are performed for the combination.

The first threshold value is not specifically limited and can be set as appropriate. For example, the first threshold value may be set in accordance with PCR conditions such as the amount of genomic DNA that is a template for polymerase chain reaction.

Here, consideration is given to a case where the first threshold value is set to "+3" in the example provided in the "local alignment" described above.

In the above example, the local alignment score is "-2" and is less than the first threshold value, that is, "+3". Thus, the combination of the base sequences with SEQ ID NOs: 1 and 2 can be determined to have low probability of primer dimer formation.

Note that this step is performed on all the combinations for which local alignment scores are calculated in the local alignment step S103, the first step of local alignment S203, the second step of local alignment S213, the first local alignment step S303, or the second local alignment step S313.

<<Global Alignment Step>>

As used herein, global alignment step S105 (Fig. 4), first step of global alignment S205 and second step of global alignment S215 (Fig. 5), and first global alignment step S305 and second global alignment step S315 (Fig. 6) are collectively referred to sometimes simply as "global alignment step".

(First aspect: global alignment step S105)

In Fig. 4, this step is represented as "global alignment".

In the first aspect, the global alignment step (e) is a step of performing pairwise global alignment, for all combinations for selecting base sequences of two candidate primers from among base sequences of candidate primers selected in the first-stage selection step, on base sequences having a preset sequence length and including 3'-ends of two base sequences included in each of the combinations, to determine global alignment scores.

(Second aspect: first step of global alignment S205 and second step of global alignment S215)

In Fig. 5, these steps are represented as "global alignment: first" and "global alignment: second".

In the second aspect, the first step of global alignment ($e_1$) is a step of performing pairwise global alignment, for all combinations for selecting base sequences of two candidate primers from among base sequences of candidate primers selected in the first step of first-stage selection, on base sequences having a preset sequence length and including 3'-ends of two base sequences included in each of the combinations, to determine global alignment scores, and the second step of global alignment ($e_2$) is a step of performing pairwise global alignment, for all combinations for selecting base sequences of two candidate primers and all combinations for selecting a base sequence of one candidate primer and a base sequence of one primer that has already been employed from among base sequences of candidate primers selected in the second step of first-stage selection and from among base sequences of primers that have already been employed, on base sequences having a preset sequence length and including 3'-ends of two base sequences included in each of the combinations, to determine global alignment scores.

(Third aspect: first global alignment step S305 and second global alignment step S315)

In Fig. 6, these steps are represented as "first global alignment" and "second global alignment".

In the third aspect, the first global alignment step (e-1) is a step of performing pairwise global alignment, for all combinations for selecting base sequences of two candidate primers from among base sequences of candidate primers selected in the first first-stage selection step, on base sequences having a preset sequence length and including 3'-ends of two base sequences included in each of the combinations, to determine global alignment scores, and the second global alignment step (e-2) is a step of performing pairwise global alignment, for all combinations for selecting base sequences of two candidate primers and all combinations for selecting a base sequence of one candidate primer and a base sequence of one primer that has already been employed from among base sequences of candidate primers selected in the second first-stage selection step and from among base sequences of primers that have already been employed, on base sequences having a preset sequence length and including 3'-ends of two base sequences included in each of the combinations, to determine global alignment scores.

(Method for global alignment)

A global alignment score is determined by extracting two primers from the group consisting of all the candidate primers generated in the "candidate primer base sequence generation step" (when the "local alignment step" and the "first-stage selection step" are performed previously, if there is a combination of candidate primers having local alignment scores less than the first threshold value, all the candidate primers included in the combination) and all the primers that have already been employed (only when there is present a primer that has already been employed) and by performing pairwise global alignment on base sequences having a preset sequence length and including the 3'-ends of the extracted primers.

A combination of base sequences to be subjected to global alignment may be a combination selected with allowed overlap or a combination selected without allowed overlap. However, if the probability of primer dimer formation between primers having the same base sequence has not yet been evaluated, it is preferable to use a combination selected with allowed overlap.

The total number of combinations is given by "${}_xH_2={}_{x+1}C_2 = (x + 1)!/2(x - 1)!$" when combinations are selected with allowed overlap, and is given by "${}_xC_2=x(x - 1)/2$" when combinations are selected without allowed overlap, where x denotes the total number of base sequences to be subjected to global alignment.

Global alignment is alignment to be performed on "entire sequences" and allows examination of the complementarity of the entire sequences.

As used here, the "entire sequence" refers to the entire base sequence having a preset sequence length and including the 3'-end of a base sequence of a candidate primer.

Note that in global alignment, a gap may be inserted. The gap refers to an insertion and/or deletion (indel) of a base.

In global alignment, furthermore, a match is determined when bases in a base sequence pair are complementary to each other, and a mismatch is determined when bases in a base sequence pair are not complementary to each other.

Alignment is performed such that a score is set for each of a match, a mismatch, and an indel and the total score is maximum. The scores may be set as appropriate. For example, scores may be set as in Table 1 above. In Table 1, "-" indicates a gap (insertion and/or deletion (indel)).

For example, consideration is given to global alignment of, for base sequences with SEQ ID NOs: 1 and 2 given in Table 5 below, three bases (indicated by capital letters) at the 3'-end of each base sequence. Here, scores are assumed to be given in Table 1.

Table 5

| | Base sequence (5' → 3') |
|---|---|
| SEQ ID NO: 1 | cttcgatgcggaccttcTGG |

(continued)

| | Base sequence (5' → 3') |
|---|---|
| SEQ ID NO: 2 | tctcccacatccgpctaTGG |

Global alignment is performed on three bases (indicated by capital letters) at the 3'-end of the base sequence with SEQ ID NO: 1 and the base sequence of three bases (indicated by capital letters) at the 3'-end of SEQ ID NO: 2 so as to obtain a maximum score, yielding alignment (pairwise alignment) given in Table 6 below. In Table 6, a mismatch is denoted by ":".

Table 6

| | | | | | |
|---|---|---|---|---|---|
| Three bases at 3'-end of SEQ ID NO: 1 | 5'- | T | G | G | -3' |
| | | : | : | : | |
| Three bases at 3'-end of SEQ ID NO: 2 | 3'- | G | G | T | -5' |

This (pairwise) alignment includes 3 mismatches and no match and indel (gap).

Thus, the global alignment score based on this (pairwise) alignment is given by $(+1) \times 0 + (-1) \times 3 + (-1) \times 0 = -3$.

Note that alignment (pairwise alignment) may be obtained using the dot matrix method, the dynamic programming method, the word method, or any of various other methods.

<<Second-Stage Selection Step>>

As used herein, second-stage selection step S106 (Fig. 4), first step of second-stage selection S206 and second step of second-stage selection S216 (Fig. 5), and first second-stage selection step S306 and second second-stage selection step S316 (Fig. 6) are collectively referred to sometimes simply as "second-stage selection step".

(First aspect: second-stage selection step S106)

In Fig. 4, this step is represented as "second-stage selection".

In the first aspect, the second-stage selection step (f) is a step of performing second-stage selection of base sequences of candidate primers for PCR amplifying the target region on the basis of the global alignment scores.

(Second aspect: first step of second-stage selection S206 and second step of second-stage selection S216)

In Fig. 5, these steps are represented as "second-stage selection: first" and "second-stage selection: second".

In the second aspect, the first step of second-stage selection ($f_1$) is a step of performing second-stage selection of base sequences of candidate primers for PCR amplifying the first target region on the basis of the global alignment scores, and the second step of second-stage selection ($f_2$) is a step of performing second-stage selection of base sequences of candidate primers for PCR amplifying the second target region on the basis of the global alignment scores.

(Third aspect: first second-stage selection step S306 and second second-stage selection step S316)

In Fig. 6, these steps are represented as "first second-stage selection" and "second second-stage selection".

In the third aspect, the first second-stage selection step (f-1) is a step of performing second-stage selection of base sequences of candidate primers for PCR amplifying the first target region on the basis of the global alignment scores, and the second second-stage selection step (f-2) is a step of performing second-stage selection of base sequences of candidate primers for PCR amplifying the second target region on the basis of the global alignment scores.

(Method for second-stage selection)

A threshold value for global alignment scores (referred to also as "second threshold value") is set in advance.

If a global alignment score is less than the second threshold value, the combination of two base sequences is determined to have low probability of dimer formation, and then the subsequent step is performed.

On the other hand, if a global alignment score is not less than the second threshold value, the combination of two base sequences is determined to have high probability of dimer formation, and no further steps are performed for the combination.

The second threshold value is not specifically limited and can be set as appropriate. For example, the second threshold value may be set in accordance with PCR conditions such as the amount of genomic DNA that is a template for polymerase chain reaction.

Note that base sequences including several bases from the 3'-ends of primers are set to be the same, whereby a global alignment score determined by performing pairwise global alignment on base sequences having a preset number of bases including the 3'-ends of the base sequences of the respective primers can be made less than the second threshold value.

Here, consideration is given to a case where the second threshold value is set to "+3" in the example provided in the "global alignment step" described above.

In the above example, the global alignment score is "-3" and is less than the second threshold value, that is, "+3". Thus, the combination of the base sequences with SEQ ID NOs: 1 and 2 can be determined to have low probability of primer dimer formation.

Note that this step is performed on all the combinations for which global alignment scores are calculated in the global alignment step S105, the first step of global alignment S205, the second step of global alignment S215, the first global alignment step S305, or the second global alignment step S315.

In addition, to reduce the amount of computation, preferably, both the "global alignment step" and the "second-stage selection step" are performed previously, and both the "local alignment step" and the "first-stage selection step" are performed on a combination of base sequences of primers that have been subjected to the "second-stage selection step". In particular, as the number of target regions and the number of base sequences of candidate primers increase, the effect of reducing the amount of computation increases, leading to an increase in the speed of the overall processing.

This is because in the "global alignment step", global alignment is performed on base sequences having a short length, that is, the "preset sequence length", which requires less computation than the calculation of a local alignment score to find partial sequences having high complementarity from the entire base sequences under the condition that the 3'-ends are included, resulting in higher-speed processing. Note that it is known that a commonly known algorithm allows global alignment to be performed at a higher speed than local alignment when the alignments are performed on sequences having the same length.

<<Amplification Sequence Length Check Step>>

A combination of base sequences of candidate primers determined to have low probability of primer dimer formation in the "first-stage selection step" and the "second-stage selection step" may be subjected to an amplification sequence length check step (not illustrated) to compute the distance between the ends of the base sequences of the candidate primers on the chromosomal DNA to determine whether the distance falls within a preset range.

If the distance between the ends of the base sequences falls within the preset range, the combination of the base sequences of the candidate primers can be determined to be likely to amplify the target region in a suitable manner. The distance between the ends of the base sequences of the candidate primers is not specifically limited and may be set as appropriate in accordance with PCR conditions such as the type of enzyme (DNA polymerase). For example, the range may be set to any of various ranges such as a range of 100 to 200 bases (pairs), a range of 120 to 180 bases (pairs), a range of 140 to 180 bases (pairs), a range of 140 to 160 bases (pairs), and a range of 160 to 180 bases (pairs).

<<Primer Employment Step>>

As used herein, primer employment step S107 (Fig. 4), first step of primer employment S207 and second step of primer employment S217 (Fig. 5), and first primer employment step S307 and second primer employment step S317 (Fig. 6) are collectively referred to sometimes simply as "primer employment step".

(First aspect: primer employment step S107)

In Fig. 4, this step is represented as "primer employment".

In the first aspect, the primer employment step (g) is a step of employing, as base sequences of primers for PCR amplifying the target region, base sequences of candidate primers selected in both the first-stage selection step and the second-stage selection step.

(Second aspect: first step of primer employment S207 and second step of primer employment S217)

In Fig. 5, these steps are represented as "primer employment: first" and "primer employment: second".

In the second aspect, the first step of primer employment ($g_1$) is a step of employing, as base sequences of primers for PCR amplifying the first target region, base sequences of candidate primers selected in both the first step of first-stage selection and the first step of second-stage selection, and the second step of primer employment ($g_2$) is a step of employing, as base sequences of primers for PCR amplifying the second target region, base sequences of candidate primers selected in both the second step of first-stage selection and the second step of second-stage selection.

(Third aspect: first primer employment step S307 and second primer employment step S317)

In Fig. 6, these steps are represented as "first primer employment" and "second primer employment".

In the third aspect, the first primer employment step (g-1) is a step of employing base sequences of candidate primers selected in both the first first-stage selection step and the first second-stage selection step as base sequences of primers for PCR amplifying the first target region, and the second primer employment step (g-2) is a step of employing base sequences of candidate primers selected in both the second first-stage selection step and the second second-stage selection step as base sequences of primers for PCR amplifying the second target region.

(Method for primer employment)

In the primer employment step, base sequences of candidate primers having a local alignment score less than the first threshold value, where the local alignment score is determined by performing pairwise local alignment on base sequences of candidate primers under the condition that the partial sequences to be compared include the 3'-ends of the base sequences, and having a global alignment score less than the second threshold value, where the global alignment score is determined by performing pairwise global alignment on base sequences having a preset number of bases including the 3'-ends of the base sequences of the candidate primers, are employed as base sequences of primers for amplifying a target region.

For example, consideration is given to the employment of base sequences with SEQ ID NOs: 1 and 2 given in Table 7 as base sequences of primers for amplifying a target region.

Table 7

| | Base sequence (5' → 3') |
|---|---|
| SEQ ID NO: 1 | CTTCGATGCGGACCTTCTGG |
| SEQ ID NO: 2 | TCTCCCACATCCGGCTATGG |

As described previously, for the combination of SEQ ID NO: 1 and SEQ ID NO: 2, the local alignment score is "-2" and is thus less than the first threshold value, that is, "+3". Further, the global alignment score is "-3" and is thus less than the second threshold value, that is, "+3".

Accordingly, the base sequence of the candidate primer indicated by SEQ ID NO: 1 and the base sequence of the candidate primer indicated by SEQ ID NO: 2 can be employed as base sequences of primers for amplifying a target region.

<<Primer Design for Other regions of interest>>

After the employment of primers for one region of interest, primers may further be designed for any other region of interest.

In the first aspect, if a base sequence of a candidate primer for any other region of interest has been generated in the candidate primer base sequence generation step S102, the local alignment step S103 and the following steps are performed. If a base sequence of a candidate primer for any other region of interest has not been generated, no region of interest has been selected in the target region selection step S101. Thus, in the target region selection step S101, any other region of interest is selected. Then, in the candidate primer base sequence generation step S102, a base sequence of a candidate primer for this region of interest is generated. After that, the local alignment step S103 and the subsequent steps are performed.

In the second aspect, the second step of target region selection S211 is repeated from the selection of a region of interest other than the first region of interest.

In the third aspect, base sequences of candidate primers for the regions of interest selected in the plurality-of-target-region selection step S301 have been generated in the plurality-of-candidate-primer-base-sequence generation step S302. Thus, the process repeats from the second local alignment step S313.

<<Feature Point in Designing of Primers, etc.>>

In brief, a feature in a method for designing primers for PCR amplifying regions of interest in a method for designing primers for multiplex PCR according to the present invention is that a plurality of specific target regions are selected, nearby base sequences are searched for, the complementarity of the found nearby base sequences to each of extracted primer sets is examined, and base sequences with low complementarity are selected to obtain a primer group in which primers are not complementary to each other and for which a target region is included in an object to be amplified.

A feature point in the examination of the complementarity of base sequences of primers is to generate a primer group so as to reduce the complementarity of the entire sequences by using local alignment and reduce the complementarity of ends of the base sequences of the primers by using global alignment.

Furthermore, as a Tm value for generating a base sequence of a candidate primer, a Tm value range calculated based on a target value and an actual value is used, thereby enabling more stable PCR amplification of a region of interest.

The present invention will be described more specifically hereinafter with reference to Examples. However, the present invention is not limited to these Examples.

[Examples]

[Example 1]

1. Primer design using typical Tm value range

Tm values were set to a typical range of 60°C to 80°C (referred to as "first Tm value range") and primers for PCR amplifying regions of interest were designed. Among the designed primers, primers for PCR amplifying regions of interest V1 to V20 are provided in Table 8.

Table 8

| Region of interest | | | Primer | | |
|---|---|---|---|---|---|
| Name | Chromosome | Start point coordinate (upper)<br>End point coordinate (lower) | Name | Base sequence (5' → 3') | SEQ ID NO: |
| V1 | 13 | 20763333 | V01-F | CTTCGATGCGGACCTTCTGG | 1 |
| | | 20763509 | V01-R | TCTCCCACATCCGGCTATGG | 2 |
| V2 | 13 | 20763795 | V02-F | GGAGACTTCTCTGAGTCTGG | 3 |
| | | 20763948 | V02-R | ACACGTTCAAGAGGGTTTGG | 4 |
| V3 | 13 | 20764098 | V03-F | CCTCTGCAGAGCTTCCTTGG | 5 |
| | | 20764260 | V03-R | CACGGTTCTCCTGTACTTGG | 6 |
| V4 | 13 | 20764701 | V04-F | AGTTCAGCGCTGAAGCTTGG | 7 |
| | | 20764874 | V04-R | CTTGTTTAGGAGAGCGTTGG | 8 |
| V5 | 13 | 20765172 | V05-F | TTTAGCTTCACTGAGCTTGG | 9 |
| | | 20765344 | V05-R | CTCGGTGGTTCTGCTGTTGG | 10 |
| V6 | 13 | 20777714 | V06-F | CACTGTTGAGTAGAGAGTGG | 11 |
| | | 20777882 | V06-R | TTCGCTTAATCTTTGGCTGG | 12 |
| V7 | 13 | 20782007 | V07-F | TCGAAATGGCATGTGTCTGG | 13 |
| | | 20782180 | V07-R | GCCTAAGAATTACCCGGTGG | 14 |
| V8 | 13 | 20822695 | V08-F | TGGATAGGCTGGATCAGTGG | 15 |
| | | 20822854 | V08-R | GAACCACAGTCAGGAGATGG | 16 |
| V9 | 13 | 20831095 | V09-F | ATCAGCAGGACTGTGCATGG | 17 |
| | | 20831251 | V09-R | TACAACCTGGCTTAGAATGG | 18 |

(continued)

| Region of interest | | | Primer | | |
|---|---|---|---|---|---|
| Name | Chromosome | Start point coordinate (upper)<br>End point coordinate (lower) | Name | Base sequence (5' → 3') | SEQ ID NO: |
| V10 | 13 | 20831937 | V10-F | GTGCCTTCTCTTCGTTCTGG | 19 |
| | | 20832088 | V10-R | TGACCCGCTTGTGTCAATGG | 20 |
| V11 | 13 | 20835554 | V11-F | TGGCCCCTACTTAAATCTGG | 21 |
| | | 20835732 | V11-R | TGCTGGAGCGAGTAGACTGG | 22 |
| V12 | 13 | 20838576 | V12-F | CTGAGTAAGTTCAGGATTGG | 23 |
| | | 20838740 | V12-R | TTCAGTTATCAGTGCAGTGG | 24 |
| V13 | 13 | 20840222 | V13-F | AGTCCCAGCACTCTCTGTGG | 25 |
| | | 20840395 | V13-R | CCCACTGGGATGCTAACTGG | 26 |
| V14 | 13 | 20846339 | V14-F | GAAAGGAACGTGTTGAGTGG | 27 |
| | | 20846499 | V14-R | CCCCTCATGATTTAAGATGG | 28 |
| V15 | 13 | 20872609 | V15-F | GGAAGCATCCAAGGAAGTGG | 29 |
| | | 20872781 | V15-R | AGCACATGCAGTGCCTGTGG | 30 |
| V16 | 13 | 20873614 | V16-F | CCACTACCACTAGGGGATGG | 31 |
| | | 20873782 | V16-R | TAGCTGCCAAAGACTGTTGG | 32 |
| V17 | 13 | 20876415 | V17-F | AACAGTGAATGGTGCATTGG | 33 |
| | | 20876565 | V17-R | AGTCTTGAGCGTGTTAGTGG | 34 |
| V18 | 13 | 20894986 | V18-F | CTCACCAAAGCTGAGACTGG | 35 |
| | | 20895160 | V18-R | TGCCTGTTGGGTTTTGCTGG | 36 |
| V19 | 13 | 20895632 | V19-F | GCAACACTAACATAGGATGG | 37 |
| | | 20895780 | V19-R | TGACTTCTGCGCAAATTTGG | 38 |
| V20 | 13 | 20914055 | V20-F | TTGTAGGAGCCTGGGCTTGG | 39 |
| | | 20914209 | V20-R | GGGGAAACACTATGAAGTGG | 40 |

2. Experimental results of primer design using typical Tm value range

Table 9 shows the number of sequence reads in regions of interest No. 1 to No. 12 in cells No. 1 to No. 5, and the coefficient of variation for each region of interest.

In the case of primer design using the first Tm value range, regions of interest for which the coefficient of variation for each region of interest exceeds 1.0 were present, and PCR amplification variations were large.

Table 9

| | | Number of sequence reads | | | | | |
|---|---|---|---|---|---|---|---|
| | | Cell | | | | | Coefficient of variation for each region of interest |
| | No. | 1 | 2 | 3 | 4 | 5 | |
| Region of interest | 1 | 1501 | 1374 | 2218 | 8077 | 864 | 1.063687 |
| | 2 | 63 | 60 | 228 | 635 | 61 | 1.187320 |
| | 3 | 252 | 208 | 538 | 2976 | 428 | 1.339199 |
| | 4 | 653 | 688 | 1530 | 4740 | 386 | 1.130052 |
| | 5 | 95 | 113 | 188 | 1417 | 8 | 1.625496 |
| | 6 | 38 | 47 | 50 | 615 | 40 | 1.617203 |
| | 7 | 65 | 77 | 126 | 1250 | 0 | 1.748873 |
| | 8 | 14 | 20 | 12 | 207 | 0 | 1.733822 |
| | 9 | 2 | 1 | 0 | 42 | 2 | 1.940724 |
| | 10 | 163 | 159 | 376 | 1675 | 11 | 1.431055 |
| | 11 | 5 | 0 | 0 | 0 | 0 | 2.236068 |
| | 12 | 17 | 7 | 2 | 0 | 0 | 1.382744 |

3. Calculation of Tm value range

A target value of the coefficient of variation for the number of sequence reads was set to 1.0, the threshold value was set to a value that is √2 times of the target value, and a new Tm value range (referred to as "second Tm value range") was calculated from the Tm value of a primer for which each region of interest was PCR amplified and from the coefficient of variation for each region of interest.

4. Primer design using new Tm value range

Tm values were set to the second Tm value range and primers for PCR amplifying regions of interest were designed. Among the designed primers, primers for PCR amplifying the regions of interest V1 and V21 to V39 are provided in Table 10.

Table 10

| Region of interest | | | Primer | | |
|---|---|---|---|---|---|
| Name | Chromosome | Start point coordinate (upper) / End point coordinate (lower) | Name | Base sequence (5' → 3') | SEQ ID NO: |
| V1 | 13 | 20763333 | V01-F | CTTCGATGCGGACCTTCTGG | 1 |
| | | 20763509 | V01-R | TCTCCCACATCCGGCTATGG | 2 |
| V21 | 13 | 21205086 | V21-F | TTTCCCCGACCATAAGCTTG | 41 |
| | | 21205235 | V21-R | ATACAGGGCTGAGAGATTGG | 42 |
| V22 | 13 | 21619945 | V22-F | TGATAAGGTCCGAACTTTGG | 43 |
| | | 21620115 | V22-R | GCGACTGCAAGAGATTCGTG | 44 |
| V23 | 13 | 23898446 | V23-F | ATTTGCTGCTGACCAGGGTG | 45 |
| | | 23898625 | V23-R | AGGTACAGCTTCCCATCTGG | 46 |
| V24 | 13 | 24797765 | V24-F | CCGTGTGTGAGATTCTCGTG | 47 |
| | | 24797943 | V24-R | ACTGCTCAGGGTCCTCTGTG | 48 |

(continued)

| Region of interest | | | Primer | | |
|---|---|---|---|---|---|
| Name | Chromosome | Start point coordinate (upper) / End point coordinate (lower) | Name | Base sequence (5' → 3') | SEQ ID NO: |
| V25 | 13 | 25009017 | V25-F | GTAAAGCCTCCAGGATGTTG | 49 |
| | | 25009170 | V25-R | CTGGCACTTGTGCTGACTGG | 50 |
| V26 | 13 | 25029140 | V26-F | CCAAAGCGCACTCACCTGTG | 51 |
| | | 25029301 | V26-R | TAGCCAGTGAGAGCGAAGTG | 52 |
| V27 | 13 | 25264984 | V27-F | GGCCTAGAGGACGATGCTTG | 53 |
| | | 25265146 | V27-R | TGTTGATAACCATGCCGGTG | 54 |
| V28 | 13 | 25266857 | V28-F | TGCTGGACAGTGACTCATGG | 55 |
| | | 25267020 | V28-R | CATTTTCCTGTCCTGGCTTG | 56 |
| V29 | 13 | 25453371 | V29-F | ATCCAGTTCATATGCCGTTG | 57 |
| | | 25453549 | V29-R | GCGTTGCTGTCATTCCTTTG | 58 |
| V30 | 13 | 26043061 | V30-F | CCTGGCGGTTGACTTCTTTG | 59 |
| | | 26043241 | V30-R | AATTTGTTGAGATGCGGTTG | 60 |
| V31 | 13 | 28367853 | V31-F | AGAAGCAGGTGAAGATCTGG | 51 |
| | | 28368020 | V31-R | CGTCATCCTCGGAGCACTTG | 52 |
| V32 | 13 | 28537192 | V32-F | AGAGTCCACGCTCCTCATGG | 53 |
| | | 28537358 | V32-R | CAGAGCCCTTGAGTCCGGTG | 54 |
| V33 | 13 | 28893594 | V33-F | AGACCACACGTCGCTCTTGG | 55 |
| | | 28893738 | V33-R | GGACACTCGGGTTGAATGTG | 56 |
| V34 | 13 | 29600265 | V34-F | GAGAGAACAAGACGGAGGTG | 57 |
| | | 29600444 | V34-R | GGAGGGGTGCTGGAATATTG | 58 |
| V35 | 13 | 29855745 | V35-F | AGATGACGGCAGTAGGATTG | 59 |
| | | 29855886 | V35-R | AGAGATGCCTTCAGAACTGG | 60 |
| V36 | 13 | 32784937 | V36-F | ACTGGCCTAGTGTTCCTGTG | 61 |
| | | 32785116 | V36-R | GTCACAATGCTGGACGATGG | 62 |
| V37 | 13 | 33704042 | V37-F | ATTTGGCCCTAGCCCTCGTG | 63 |
| | | 33704222 | V37-R | ATTCACAGCGAAAGCAGTGG | 64 |
| V38 | 13 | 36384994 | V38-F | GAGCCACGTATGTTGGGGTG | 65 |
| | | 36385161 | V38-R | AAAGGGCTTTTGAGCTCTTG | 66 |
| V39 | 13 | 36686005 | V39-F | CCTGTTTCCCATCCAACGTG | 67 |
| | | 36686167 | V39-R | GTCACCATCATCAGAAGTGG | 68 |

5. Experimental results of primer design using new Tm value range

Table 11 shows the number of sequence reads in regions of interest No. 1 to No. 12 in cells No. 6 to No. 10, and the coefficient of variation for each region of interest.

In the case of primer design using the second Tm value range, no targeted region for which the coefficient of variation for each region of interest exceeds 1.0 is present, and PCR amplification variations were small.

Table 11

| | | Number of sequence reads | | | | | |
|---|---|---|---|---|---|---|---|
| | | Cell | | | | | Coefficient of variation for each region of interest |
| | No. | 6 | 7 | 8 | 9 | 10 | |
| Region of interest | 13 | 177 | 88 | 125 | 98 | 94 | 0.315457 |
| | 14 | 245 | 119 | 143 | 120 | 197 | 0.333031 |
| | 15 | 56 | 52 | 53 | 5 | 93 | 0.603324 |
| | 16 | 88 | 36 | 154 | 27 | 241 | 0.818340 |
| | 17 | 45 | 17 | 46 | 43 | 66 | 0.401940 |
| | 18 | 908 | 1050 | 879 | 878 | 991 | 0.081060 |
| | 19 | 62 | 1 | 132 | 84 | 70 | 0.674449 |
| | 20 | 1 | 2 | 93 | 62 | 76 | 0.914245 |
| | 21 | 143 | 206 | 189 | 110 | 146 | 0.242562 |
| | 22 | 117 | 81 | 90 | 98 | 135 | 0.208653 |
| | 23 | 108 | 95 | 99 | 123 | 51 | 0.283068 |
| | 24 | 168 | 182 | 132 | 205 | 57 | 0.388084 |
| | 25 | 113 | 179 | 84 | 130 | 103 | 0.295988 |
| | 26 | 75 | 164 | 148 | 142 | 74 | 0.355276 |
| | 27 | 228 | 219 | 143 | 237 | 115 | 0.294517 |
| | 28 | 216 | 135 | 131 | 154 | 118 | 0.256375 |
| | 29 | 46 | 33 | 38 | 32 | 23 | 0.245463 |
| | 30 | 255 | 181 | 147 | 162 | 48 | 0.469441 |
| | 31 | 112 | 117 | 165 | 88 | 31 | 0.475719 |
| | 32 | 66 | 67 | 111 | 139 | 61 | 0.389321 |
| | 33 | 113 | 65 | 56 | 94 | 39 | 0.405825 |

Reference Signs List

11 arithmetic means (CPU)
12 storage means (memory)
13 auxiliary storage means (storage)
14 input means (keyboard)
15 auxiliary input means (mouse)
16 display means (monitor)
17 output means (printer)

## Claims

1. A method for designing primers for multiplex PCR from a single cell, comprising a Tm value range setting step of setting a Tm value range for primer design, wherein
in a case where an attempt to PCR amplify m regions of interest out of n regions of interest and to count the number of sequence reads in each region of interest is made N times to calculate a coefficient of variation for the number of sequence reads in each region of interest, given that an actual value of a coefficient of variation for the number of sequence reads in an i-th region of interest is denoted by $CV_i$ and an average Tm value of a pair of primers used to PCR amplify the i-th region of interest is denoted by $Tm_i$,
in the Tm value range setting step, a Tm value range of a primer is determined by:

    a step of inputting a target value $CV_0$ of coefficients of variation for the numbers of sequence reads from input means and storing the target value $CV_0$ in storage means;
    a step of inputting the number of regions of interest m in the attempt made N times, the actual value $CV_i$ of the coefficient of variation for the number of sequence reads in the i-th region of interest, and the average Tm value $Tm_i$ of the pair of primers used to PCR amplify the i-th region of interest, and storing the number of regions of interest m, the actual value $CV_i$, and the average Tm value $Tm_i$ in the storage means;
    a step of, by arithmetic means, calculating a threshold value $CV_t$ for the coefficients of variation for the numbers of sequence reads as a function of the target value $CV_0$ in accordance with $CV_t = H(CV_0)$, and storing the threshold value $CV_t$ in the storage means;
    a step of, by the arithmetic means, separating the m regions of interest into an R1 group constituted by $m_1$ regions of interest in which the coefficient of variation $CV_i$ for the number of sequence reads satisfies $CV_i \geq CV_t$ and an R2 group constituted by $m_2$ regions of interest in which the coefficient of variation $CV_i$ for the number of sequence reads satisfies $CV_i < CV_t$, generating respective histograms for the R1 group and the R2 group, each histogram having a horizontal axis representing an average Tm value of a pair of primers used to PCR amplify each region of interest and a vertical axis representing the number of regions of interest, and storing the histograms in the storage means;
    a step of, by the arithmetic means, calculating a value designated in advance from a value at a left end of the histogram for the R1 group, a value at a right end of the histogram for the R1 group, a mode of the histogram for the R1 group, a value at a left end of the histogram for the R2 group, and a Tm value at an intersection of the histogram for the R1 group and the histogram for the R2 group, and storing the calculated value as a lower limit value of the Tm value range in the storage means;
    a step of, by the arithmetic means, calculating a value at a right end of the histogram for the R2 group, and storing the calculated value as an upper limit value of the Tm value range in the storage means; and
    a step of, by the arithmetic means, reading the lower limit value and the upper limit value stored in the storage means and displaying the lower limit value and the upper limit value on display means,
    where n is an integer satisfying $2 \leq n$, m is an integer satisfying $2 \leq m \leq n$, N is an integer satisfying $3 \leq n$, i is an integer satisfying $1 \leq i \leq m$, and $m_1$ and $m_2$ are integers satisfying $1 \leq m_1 < m$, $1 \leq m_2 < m$, and $m_1 + m_2 = m$.

2. The method for designing primers for multiplex PCR according to claim 1, wherein $CV_t = H(CV_0) = \sqrt{2} \times CV_0$ is satisfied.

3. The method for designing primers for multiplex PCR according to claim 1, wherein $CV_t = H(CV_0) = CV_0$ is satisfied.

FIG. 1

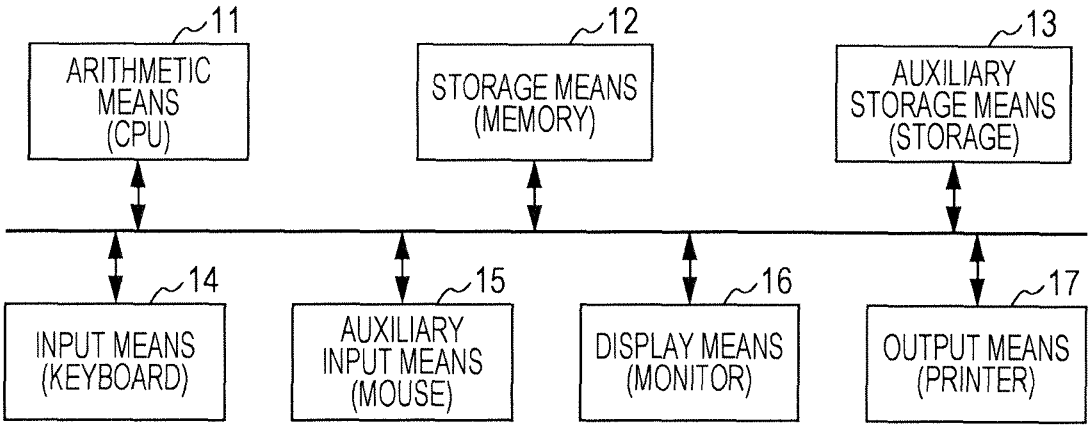
11
ARITHMETIC MEANS (CPU)
12
STORAGE MEANS (MEMORY)
13
AUXILIARY STORAGE MEANS (STORAGE)
14
INPUT MEANS (KEYBOARD)
15
AUXILIARY INPUT MEANS (MOUSE)
16
DISPLAY MEANS (MONITOR)
17
OUTPUT MEANS (PRINTER)

FIG. 2

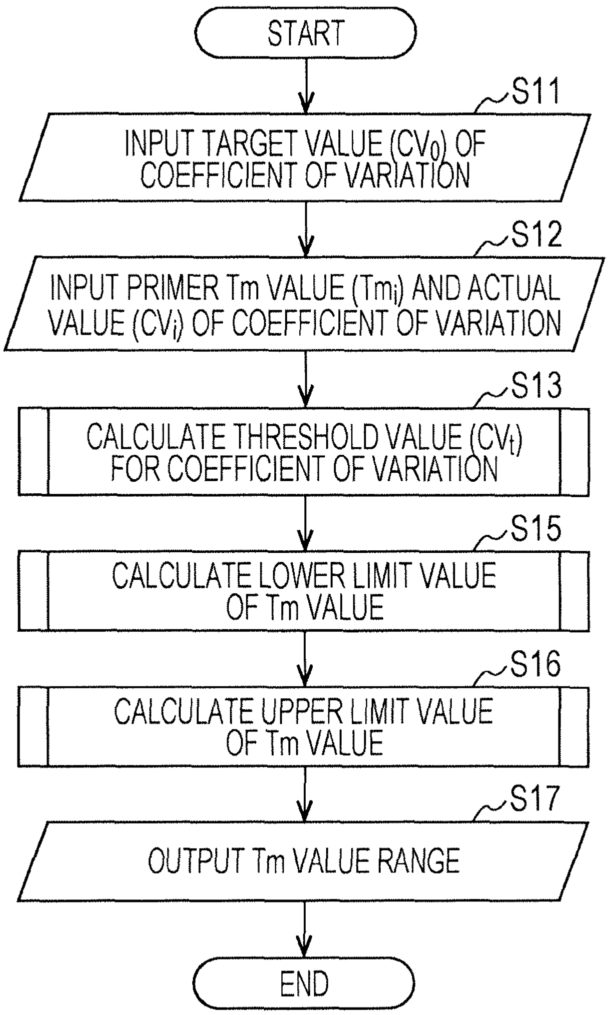
START
S11
INPUT TARGET VALUE ($CV_0$) OF COEFFICIENT OF VARIATION
S12
INPUT PRIMER Tm VALUE ($Tm_i$) AND ACTUAL VALUE ($CV_i$) OF COEFFICIENT OF VARIATION
S13
CALCULATE THRESHOLD VALUE ($CV_t$) FOR COEFFICIENT OF VARIATION
S15
CALCULATE LOWER LIMIT VALUE OF Tm VALUE
S16
CALCULATE UPPER LIMIT VALUE OF Tm VALUE
S17
OUTPUT Tm VALUE RANGE
END

FIG. 3

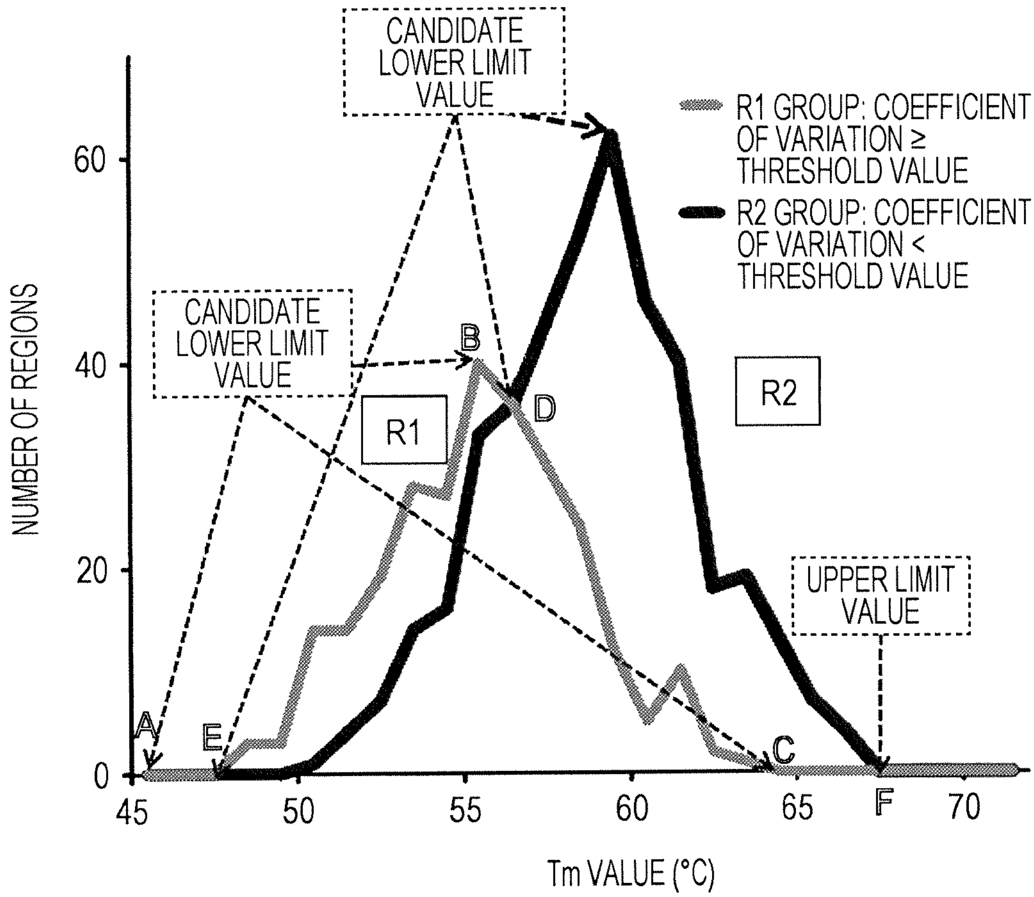
CANDIDATE LOWER LIMIT VALUE
CANDIDATE LOWER LIMIT VALUE
R1 GROUP: COEFFICIENT OF VARIATION ≥ THRESHOLD VALUE
R2 GROUP: COEFFICIENT OF VARIATION < THRESHOLD VALUE
UPPER LIMIT VALUE
R1
R2
A
B
C
D
E
F
NUMBER OF REGIONS
0
20
40
60
45
50
55
60
65
70
Tm VALUE (°C)

FIG. 4

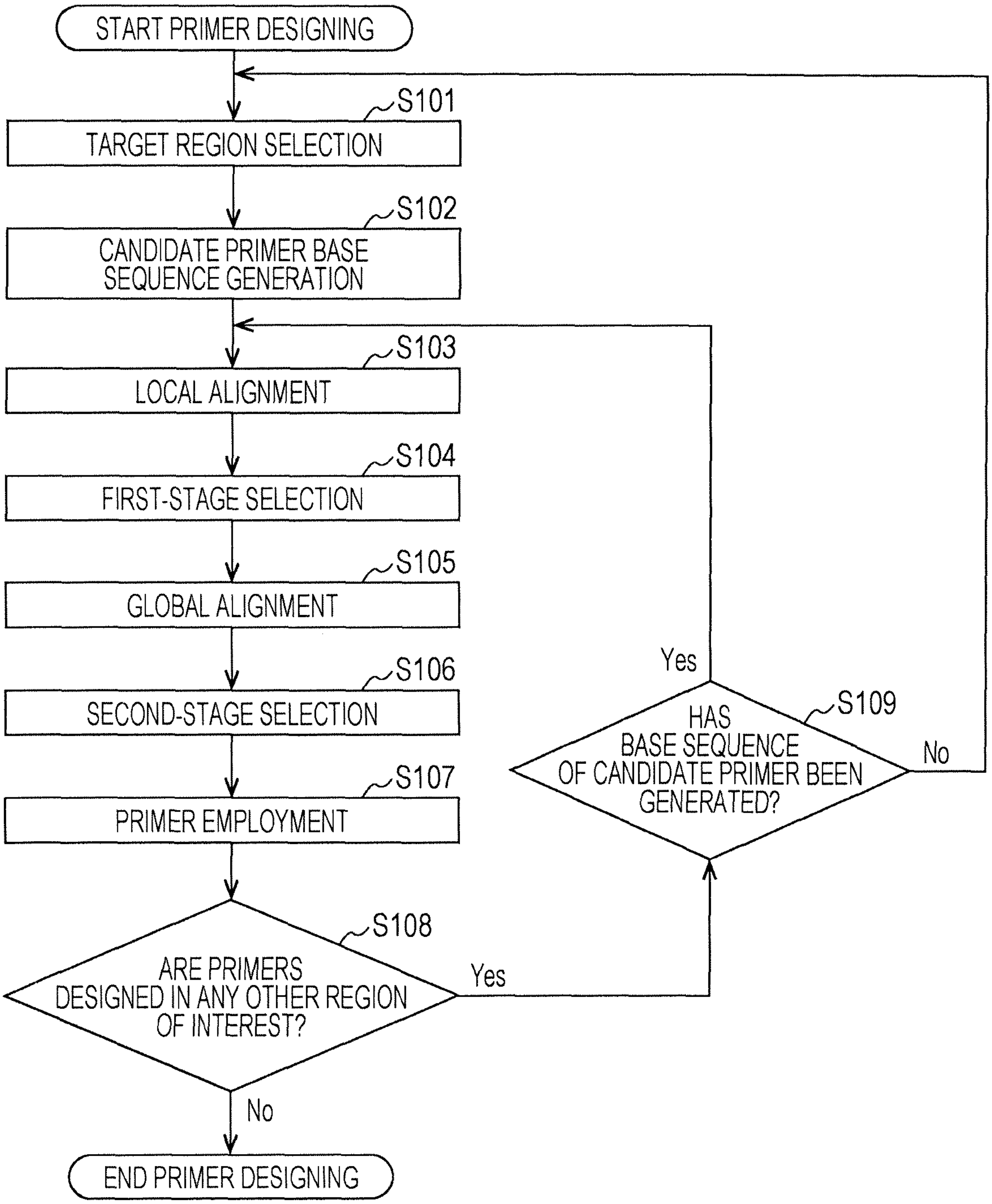
START PRIMER DESIGNING
S101
TARGET REGION SELECTION
S102
CANDIDATE PRIMER BASE SEQUENCE GENERATION
S103
LOCAL ALIGNMENT
S104
FIRST-STAGE SELECTION
S105
GLOBAL ALIGNMENT
S106
SECOND-STAGE SELECTION
S107
PRIMER EMPLOYMENT
S108
ARE PRIMERS DESIGNED IN ANY OTHER REGION OF INTEREST?
Yes
No
END PRIMER DESIGNING
S109
HAS BASE SEQUENCE OF CANDIDATE PRIMER BEEN GENERATED?
Yes
No

FIG. 5

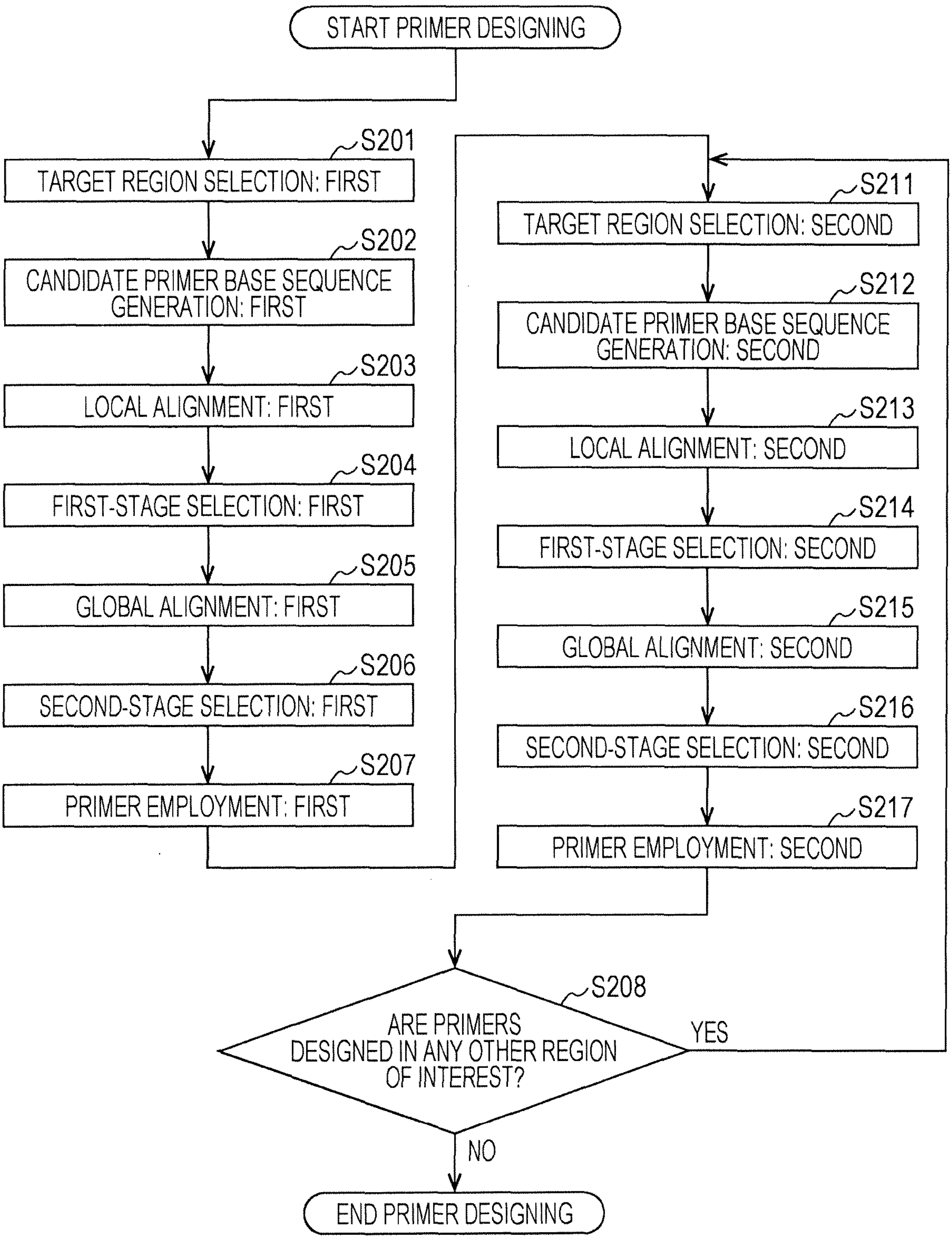
START PRIMER DESIGNING
S201
TARGET REGION SELECTION: FIRST
S202
CANDIDATE PRIMER BASE SEQUENCE GENERATION: FIRST
S203
LOCAL ALIGNMENT: FIRST
S204
FIRST-STAGE SELECTION: FIRST
S205
GLOBAL ALIGNMENT: FIRST
S206
SECOND-STAGE SELECTION: FIRST
S207
PRIMER EMPLOYMENT: FIRST
S211
TARGET REGION SELECTION: SECOND
S212
CANDIDATE PRIMER BASE SEQUENCE GENERATION: SECOND
S213
LOCAL ALIGNMENT: SECOND
S214
FIRST-STAGE SELECTION: SECOND
S215
GLOBAL ALIGNMENT: SECOND
S216
SECOND-STAGE SELECTION: SECOND
S217
PRIMER EMPLOYMENT: SECOND
S208
ARE PRIMERS DESIGNED IN ANY OTHER REGION OF INTEREST?
YES
NO
END PRIMER DESIGNING

FIG. 6

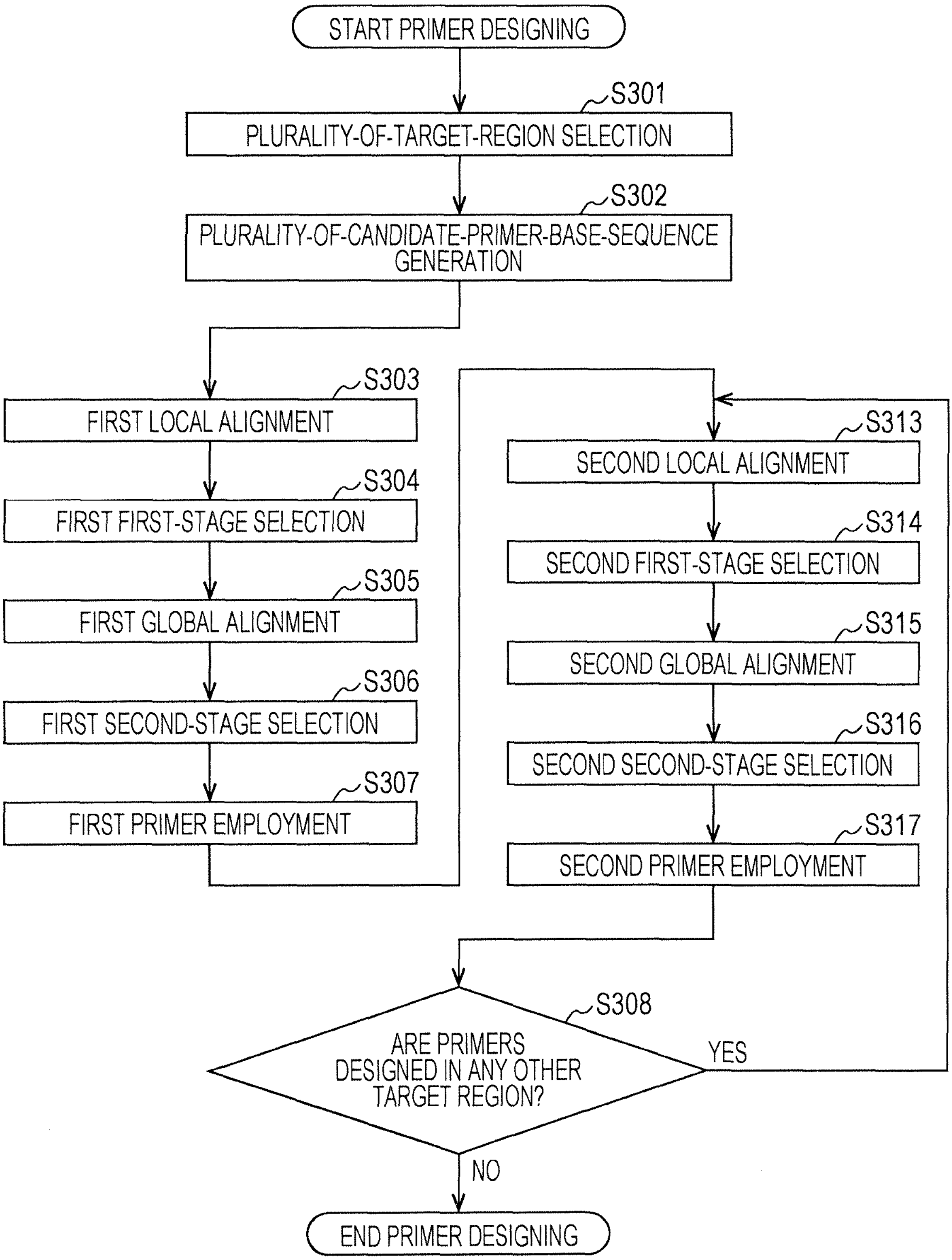
START PRIMER DESIGNING
S301
PLURALITY-OF-TARGET-REGION SELECTION
S302
PLURALITY-OF-CANDIDATE-PRIMER-BASE-SEQUENCE GENERATION
S303
FIRST LOCAL ALIGNMENT
S304
FIRST FIRST-STAGE SELECTION
S305
FIRST GLOBAL ALIGNMENT
S306
FIRST SECOND-STAGE SELECTION
S307
FIRST PRIMER EMPLOYMENT
S313
SECOND LOCAL ALIGNMENT
S314
SECOND FIRST-STAGE SELECTION
S315
SECOND GLOBAL ALIGNMENT
S316
SECOND SECOND-STAGE SELECTION
S317
SECOND PRIMER EMPLOYMENT
S308
ARE PRIMERS DESIGNED IN ANY OTHER TARGET REGION?
YES
NO
END PRIMER DESIGNING

## INTERNATIONAL SEARCH REPORT

International application No. PCT/JP2017/032252

A. CLASSIFICATION OF SUBJECT MATTER
C12Q1/68(2006.01)i, C12N15/09(2006.01)n

According to International Patent Classification (IPC) or to both national classification and IPC

B. FIELDS SEARCHED

Minimum documentation searched (classification system followed by classification symbols)
C12Q1/68, C12N15/09

Documentation searched other than minimum documentation to the extent that such documents are included in the fields searched

| | |
|---|---|
| Published examined utility model applications of Japan | 1922-1996 |
| Published unexamined utility model applications of Japan | 1971-2017 |
| Registered utility model specifications of Japan | 1996-2017 |
| Published registered utility model applications of Japan | 1994-2017 |

Electronic data base consulted during the international search (name of data base and, where practicable, search terms used)
JSTPlus/JMEDPlus/JST7580(JDreamIII), CAplus/MEDLINE/EMBASE/BIOSIS(STN), DWPI (Thomson Innovation)

C. DOCUMENTS CONSIDERED TO BE RELEVANT

| Category* | Citation of document, with indication, where appropriate, of the relevant passages | Relevant to claim No. |
|---|---|---|
| A | WO 2008/004691 A1 (SHIMADZU CORPORATION) 10 January 2008, claims, embodiments & US 2010/0070452 A1, claims, embodiments & CN 101484898 A | 1-3 |
| A | CHEN, Y. F. et al., Design of multiplex PCR primers using heuristic algorithm for sequential deletion applications, Comput. Biol. Chem., 2009, vol. 33, issue 2, pp. 181-188, ISSN 1476-9271, especially Abstract, pp. 183-184 | 1-3 |
| A | JP 2005-095025 A (SHIMADZU CORPORATION) 14 April 2055 claims, paragraphs [0005], [0015]-[0018] (Family: none) | 1-3 |

☒ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| | |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2017/032252

C (Continuation). DOCUMENTS CONSIDERED TO BE RELEVANT

| Category* | Citation of document, with indication, where appropriate, of the relevant passages | Relevant to claim No. |
|---|---|---|
| A | HYSOM, D. A. et al., Skip the Alignment: Degenerate, Multiplex Primer and Probe Design Using K-mer Matching Instead of Alignments, PLoS ONE, 2012, vol. 7, issue 4, e34560, ISSN 1932-6203, especially Abstract, p. 6, fig. 2. | 1-3 |
| A | JP 2008-212083 A (KYOTO UNIVERSITY) 18 September 2008 paragraphs [0062], [0063], fig. 2 (Family: none) | 1-3 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5079694 B


### Non-patent literature cited in the description


- **ALTSCHUL, S. A.** Basic Local Alignment Search Tool. *Journal of Molecular Biology,* October 1990, vol. 215, 403-410
- Improved tools for biological sequence comparison. **PEARSON, W. R.** Proceedings of the National Academy of Sciences of the United States of America. National Academy of Sciences of the United States of America, April 1988, vol. 85, 2444-2448